# EUROPEAN PATENT APPLICATION

(11) **EP 3 117 830 A1**
(43) Date of publication of application: **18.01.2017**
(21) Application number: 15177175.5
(22) Date of filing: 16.07.2015
(51) Int. Cl.: A61K 38/01, C07K 14/415

(54) **ANTIBACTERIAL PEPTIDES, AND USES THEREOF**

(71) Applicant: Nuritas Limited, Co. Dublin (IE)
(72) Inventor: KHALDI, Nora, Co. Dublin (IE); LOPEZ, Cyril, Dublin, 6W (IE)
(74) Representative: Purdy, Hugh Barry

(57) **Abstract**

A natural antibacterial peptide comprising a antibacterial fragment of a protein selected from SEQUENCE ID NO's: 1 to 6, and a composition comprising a plurality of antibacterial peptides, is described. Also disclosed is the use of the peptides and compositions as an antibacterial agent, or as an active agent in the treatment or prevention of diseases or conditions caused by bacterial infection.

SEQUENCE LISTING

<110>  NURITAS LIMITED

<120>  Antibacterial peptides, and uses thereof

<130>  P11686EP00

<160>  53

<170>  PatentIn version 3.5

<210>  1
<211>  459
<212>  PRT
<213>  Pisum sativum

<400>  1

Met Ala Ala Thr Thr Met Lys Ala Ser Phe Pro Leu Leu Met Leu Met
1               5                   10                  15

Gly Ile Ser Phe Leu Ala Ser Val Cys Val Ser Ser Arg Ser Asp Pro
            20                  25                  30

Gln Asn Pro Phe Ile Phe Lys Ser Asn Lys Phe Gln Thr Leu Phe Glu
        35                  40                  45

Asn Glu Asn Gly His Ile Arg Leu Leu Gln Lys Phe Asp Gln Arg Ser
    50                  55                  60

Lys Ile Phe Glu Asn Leu Gln Asn Tyr Arg Leu Leu Glu Tyr Lys Ser
65                  70                  75                  80

Lys Pro His Thr Ile Phe Leu Pro Gln His Thr Asp Ala Asp Tyr Ile
                85                  90                  95

Leu Val Val Leu Ser Gly Lys Ala Ile Leu Thr Val Leu Lys Pro Asp
            100                 105                 110

Asp Arg Asn Ser Phe Asn Leu Glu Arg Gly Asp Thr Ile Lys Leu Pro
        115                 120                 125

Ala Gly Thr Ile Ala Tyr Leu Val Asn Arg Asp Asp Asn Glu Glu Leu
    130                 135                 140

Arg Val Leu Asp Leu Ala Ile Pro Val Asn Arg Pro Gly Gln Leu Gln
145                 150                 155                 160

Ser Phe Leu Leu Ser Gly Asn Gln Asn Gln Gln Asn Tyr Leu Ser Gly
                165                 170                 175

Phe Ser Lys Asn Ile Leu Glu Ala Ser Phe Asn Thr Asp Tyr Glu Glu
            180                 185                 190

Ile Glu Lys Val Leu Leu Glu Glu His Glu Lys Glu Thr Gln His Arg
        195                 200                 205

Arg Ser Leu Lys Asp Lys Arg Gln Gln Ser Gln Glu Glu Asn Val Ile
    210                 215                 220

Val Lys Leu Ser Arg Gly Gln Ile Glu Glu Leu Ser Lys Asn Ala Lys
225                 230                 235                 240

Ser Thr Ser Lys Lys Ser Val Ser Ser Glu Ser Glu Pro Phe Asn Leu
                245                 250                 255

Arg Ser Arg Gly Pro Ile Tyr Ser Asn Glu Phe Gly Lys Phe Phe Glu
            260                 265                 270

Ile Thr Pro Glu Lys Asn Pro Gln Leu Gln Asp Leu Asp Ile Phe Val
        275                 280                 285

Asn Ser Val Glu Ile Lys Glu Gly Ser Leu Leu Leu Pro His Tyr Asn
    290                 295                 300

Ser Arg Ala Ile Val Ile Val Thr Val Asn Glu Gly Lys Gly Asp Phe
305                 310                 315                 320

Glu Leu Val Gly Gln Arg Asn Glu Asn Gln Gln Glu Gln Arg Lys Glu
                325                 330                 335

Asp Asp Glu Glu Glu Glu Gln Gly Glu Glu Glu Ile Asn Lys Gln Val
            340                 345                 350

Gln Asn Tyr Lys Ala Lys Leu Ser Ser Gly Asp Val Phe Val Ile Pro
        355                 360                 365

Ala Gly His Pro Val Ala Val Lys Ala Ser Ser Asn Leu Asp Leu Leu
    370                 375                 380

Gly Phe Gly Ile Asn Ala Glu Asn Asn Gln Arg Asn Phe Leu Ala Gly
385                 390                 395                 400

Asp Glu Asp Asn Val Ile Ser Gln Ile Gln Arg Pro Val Lys Glu Leu
                405                 410                 415

Ala Phe Pro Gly Ser Ala Gln Glu Val Asp Arg Ile Leu Glu Asn Gln
            420                 425                 430

Lys Gln Ser His Phe Ala Asp Ala Gln Pro Gln Gln Arg Glu Arg Gly
        435                 440                 445

Ser Arg Glu Thr Arg Asp Arg Leu Ser Ser Val
    450                 455

<210>  2
<211>  613
<212>  PRT
<213>  Pisum sativum

<400>  2

Met Ala Thr Thr Ile Lys Ser Arg Phe Pro Leu Leu Leu Leu Leu Gly
1               5                   10                  15

Ile Ile Phe Leu Ala Ser Val Val Cys Val Thr Tyr Ala Asn Tyr Asp
            20                  25                  30

Glu Gly Ser Glu Pro Arg Val Pro Ala Gln Arg Glu Arg Gly Arg Gln
        35                  40                  45

Glu Gly Glu Lys Glu Glu Lys Arg His Gly Glu Trp Arg Pro Ser Tyr
    50                  55                  60

Glu Lys Glu Glu Asp Glu Glu Glu Gly Gln Arg Glu Arg Gly Arg Gln
65                  70                  75                  80

Glu Gly Glu Lys Glu Glu Lys Arg His Gly Glu Trp Arg Pro Ser Tyr
                85                  90                  95

Glu Lys Gln Glu Asp Glu Glu Glu Lys Gln Lys Tyr Arg Tyr Gln Arg
            100                 105                 110

Glu Lys Glu Asp Glu Glu Glu Lys Gln Lys Tyr Gln Tyr Gln Arg Glu
        115                 120                 125

Lys Lys Glu Gln Lys Glu Val Gln Pro Gly Arg Glu Arg Trp Glu Arg
    130                 135                 140

Glu Glu Asp Glu Glu Gln Val Asp Glu Glu Trp Arg Gly Ser Gln Arg
145                 150                 155                 160

Arg Glu Asp Pro Glu Glu Arg Ala Arg Leu Arg His Arg Glu Glu Arg
                165                 170                 175

Thr Lys Arg Asp Arg Arg His Gln Arg Glu Gly Glu Glu Glu Glu Arg
            180                 185                 190

Ser Ser Glu Ser Gln Glu Arg Arg Asn Pro Phe Leu Phe Lys Ser Asn
        195                 200                 205

Lys Phe Leu Thr Leu Phe Glu Asn Glu Asn Gly His Ile Arg Leu Leu
    210                 215                 220

Gln Arg Phe Asp Lys Arg Ser Asp Leu Phe Glu Asn Leu Gln Asn Tyr
225                 230                 235                 240

Arg Leu Val Glu Tyr Arg Ala Lys Pro His Thr Ile Phe Leu Pro Gln
                245                 250                 255

His Ile Asp Ala Asp Leu Ile Leu Val Val Leu Ser Gly Lys Ala Ile
            260                 265                 270

Leu Thr Val Leu Ser Pro Asn Asp Arg Asn Ser Tyr Asn Leu Glu Arg
        275                 280                 285

Gly Asp Thr Ile Lys Leu Pro Ala Gly Thr Thr Ser Tyr Leu Val Asn
    290                 295                 300

Gln Asp Asp Glu Glu Asp Leu Arg Leu Val Asp Leu Val Ile Pro Val
305                 310                 315                 320

Asn Gly Pro Gly Lys Phe Glu Ala Phe Asp Leu Ala Lys Asn Lys Asn
                325                 330                 335

Gln Tyr Leu Arg Gly Phe Ser Lys Asn Ile Leu Glu Ala Ser Tyr Asn
            340                 345                 350

Thr Arg Tyr Glu Thr Ile Glu Lys Val Leu Leu Glu Glu Gln Glu Lys
        355                 360                 365

Asp Arg Lys Arg Arg Gln Gln Gly Glu Glu Thr Asp Ala Ile Val Lys
    370                 375                 380

Val Ser Arg Glu Gln Ile Glu Glu Leu Lys Lys Leu Ala Lys Ser Ser
385                 390                 395                 400

Ser Lys Lys Ser Leu Pro Ser Glu Phe Glu Pro Ile Asn Leu Arg Ser
                405                 410                 415

His Lys Pro Glu Tyr Ser Asn Lys Phe Gly Lys Leu Phe Glu Ile Thr
            420                 425                 430

Pro Glu Lys Lys Tyr Pro Gln Leu Gln Asp Leu Asp Leu Phe Val Ser
        435                 440                 445

Cys Val Glu Ile Asn Glu Gly Ala Leu Met Leu Pro His Tyr Asn Ser
    450                 455                 460

Arg Ala Ile Val Val Leu Leu Val Asn Glu Gly Lys Gly Asn Leu Glu
465                 470                 475                 480

Leu Leu Gly Leu Lys Asn Glu Gln Gln Glu Arg Glu Asp Arg Lys Glu
                485                 490                 495

Arg Asn Asn Glu Val Gln Arg Tyr Glu Ala Arg Leu Ser Pro Gly Asp
            500                 505                 510

Val Val Ile Ile Pro Ala Gly His Pro Val Ala Ile Thr Ala Ser Ser
        515                 520                 525

Asn Leu Asn Leu Leu Gly Phe Gly Ile Asn Ala Glu Asn Asn Glu Arg
    530                 535                 540

Asn Phe Leu Ser Gly Ser Asp Asp Asn Val Ile Ser Gln Ile Glu Asn
545                 550                 555                 560

Pro Val Lys Glu Leu Thr Phe Pro Gly Ser Val Gln Glu Ile Asn Arg
                565                 570                 575

Leu Ile Lys Asn Gln Lys Gln Ser His Phe Ala Asn Ala Glu Pro Glu
            580                 585                 590

Gln Lys Glu Gln Gly Ser Gln Gly Lys Arg Ser Pro Leu Ser Ser Ile
        595                 600                 605

Leu Gly Thr Phe Tyr
    610

<210>  3
<211>  438
<212>  PRT
<213>  Pisum sativum

<400>  3

Met Ala Ala Thr Pro Ile Lys Pro Leu Met Leu Leu Ala Ile Ala Phe
1               5                   10                  15

Leu Ala Ser Val Cys Val Ser Ser Arg Ser Asp Gln Glu Asn Pro Phe
            20                  25                  30

Ile Phe Lys Ser Asn Arg Phe Gln Thr Leu Tyr Glu Asn Glu Asn Gly
        35                  40                  45

His Ile Arg Leu Leu Gln Lys Phe Asp Lys Arg Ser Lys Ile Phe Glu
    50                  55                  60

Asn Leu Gln Asn Tyr Arg Leu Leu Glu Tyr Lys Ser Lys Pro His Thr
65                  70                  75                  80

Leu Phe Leu Pro Gln Tyr Thr Asp Ala Asp Phe Ile Leu Val Val Leu
                85                  90                  95

Asn Gly Lys Ala Thr Leu Thr Val Leu Lys Ser Asn Asp Arg Asn Ser
            100                 105                 110

Phe Asn Leu Glu Arg Gly Asp Thr Ile Lys Leu Pro Ala Gly Thr Ile
        115                 120                 125

Ala Tyr Leu Ala Asn Arg Asp Asp Asn Glu Asp Leu Arg Val Leu Asp
    130                 135                 140

Leu Ala Ile Pro Val Asn Lys Pro Gly Gln Leu Gln Ser Phe Leu Leu
145                 150                 155                 160

Ser Gly Thr Gln Asn Gln Pro Ser Leu Leu Ser Gly Phe Ser Lys Asn
                165                 170                 175

Ile Leu Glu Ala Ala Phe Asn Thr Asn Tyr Glu Glu Ile Glu Lys Val
            180                 185                 190

Leu Leu Glu Gln Gln Glu Gln Glu Pro Gln His Arg Arg Ser Leu Lys
        195                 200                 205

Asp Arg Arg Gln Glu Ile Asn Glu Glu Asn Val Ile Val Lys Val Ser
    210                 215                 220

Arg Glu Gln Ile Glu Glu Leu Ser Lys Asn Ala Lys Ser Ser Ser Lys
225                 230                 235                 240

Lys Ser Val Ser Ser Glu Ser Gly Pro Phe Asn Leu Arg Ser Arg Asn
                245                 250                 255

Pro Ile Tyr Ser Asn Lys Phe Gly Lys Phe Phe Glu Ile Thr Pro Glu
            260                 265                 270

Lys Asn Gln Gln Leu Gln Asp Leu Asp Ile Phe Val Asn Ser Val Asp
        275                 280                 285

Ile Lys Glu Gly Ser Leu Leu Leu Pro Asn Tyr Asn Ser Arg Ala Ile

290                 295                 300

Val Ile Val Thr Val Thr Glu Gly Lys Gly Asp Phe Glu Leu Val Gly
305                 310                 315                 320

Gln Arg Asn Glu Asn Gln Gly Lys Glu Asn Asp Lys Glu Glu Glu Gln
                325                 330                 335

Glu Glu Glu Thr Ser Lys Gln Val Gln Leu Tyr Lys Ala Lys Leu Ser
            340                 345                 350

Pro Gly Asp Val Phe Val Ile Pro Ala Gly His Pro Val Ala Ile Asn
        355                 360                 365

Ala Ser Ser Asp Leu Asn Leu Ile Gly Phe Gly Ile Asn Ala Glu Asn
    370                 375                 380

Asn Glu Arg Asn Phe Leu Ala Gly Glu Glu Asp Asn Val Ile Ser Gln
385                 390                 395                 400

Val Gln Arg Pro Val Lys Glu Leu Ala Phe Pro Gly Ser Ser His Glu
                405                 410                 415

Ile Asp Arg Leu Leu Lys Asn Gln Lys Gln Ser Tyr Phe Ala Asn Ala
            420                 425                 430

Gln Pro Leu Gln Arg Glu
        435

<210>  4
<211>  499
<212>  PRT
<213>  Oryza sativa

<400>  4

Met Ala Ser Ser Val Phe Ser Arg Phe Ser Ile Tyr Phe Cys Val Leu
1               5                   10                  15

Leu Leu Cys His Gly Ser Met Ala Gln Leu Phe Asn Pro Ser Thr Asn
            20                  25                  30

Pro Trp His Ser Pro Arg Gln Gly Ser Phe Arg Glu Cys Arg Phe Asp
        35                  40                  45

Arg Leu Gln Ala Phe Glu Pro Leu Arg Lys Val Arg Ser Glu Ala Gly
    50                  55                  60

Val Thr Glu Tyr Phe Asp Glu Lys Asn Glu Leu Phe Gln Cys Thr Gly

65                  70                  75                  80

Thr Phe Val Ile Arg Arg Val Ile Gln Pro Gln Gly Leu Leu Val Pro
                85                  90                  95

Arg Tyr Thr Asn Ile Pro Gly Val Val Tyr Ile Ile Gln Gly Arg Gly
            100                 105                 110

Ser Met Gly Leu Thr Phe Pro Gly Cys Pro Ala Thr Tyr Gln Gln Gln
        115                 120                 125

Phe Gln Gln Phe Ser Ser Gln Gly Gln Ser Gln Ser Gln Lys Phe Arg
    130                 135                 140

Asp Glu His Gln Lys Ile His Gln Phe Arg Gln Gly Asp Ile Val Ala
145                 150                 155                 160

Leu Pro Ala Gly Val Ala His Trp Phe Tyr Asn Asp Gly Asp Ala Pro
                165                 170                 175

Ile Val Ala Val Tyr Val Tyr Asp Val Asn Asn Asn Ala Asn Gln Leu
            180                 185                 190

Glu Pro Arg Gln Lys Glu Phe Leu Leu Ala Gly Asn Asn Asn Arg Ala
        195                 200                 205

Gln Gln Gln Gln Val Tyr Gly Ser Ser Ile Glu Gln His Ser Gly Gln
    210                 215                 220

Asn Ile Phe Ser Gly Phe Gly Val Glu Met Leu Ser Glu Ala Leu Gly
225                 230                 235                 240

Ile Asn Ala Val Ala Ala Lys Arg Leu Gln Ser Gln Asn Asp Gln Arg
                245                 250                 255

Gly Glu Ile Ile His Val Lys Asn Gly Leu Gln Leu Leu Lys Pro Thr
            260                 265                 270

Leu Thr Gln Gln Gln Glu Gln Ala Gln Ala Gln Asp Gln Tyr Gln Gln
        275                 280                 285

Val Gln Tyr Ser Glu Arg Gln Gln Thr Ser Ser Arg Trp Asn Gly Leu
    290                 295                 300

Glu Glu Asn Phe Cys Thr Ile Lys Val Arg Val Asn Ile Glu Asn Pro
305                 310                 315                 320

Ser Arg Ala Asp Ser Tyr Asn Pro Arg Ala Gly Arg Ile Thr Ser Val
                325                 330                 335

Asn Ser Gln Lys Phe Pro Ile Leu Asn Leu Ile Gln Met Ser Ala Thr
            340                 345                 350

Arg Val Asn Leu Tyr Gln Asn Ala Ile Leu Ser Pro Phe Trp Asn Val
        355                 360                 365

Asn Ala His Ser Leu Val Tyr Met Ile Gln Gly Arg Ser Arg Val Gln
    370                 375                 380

Val Val Ser Asn Phe Gly Lys Thr Val Phe Asp Gly Val Leu Arg Pro
385                 390                 395                 400

Gly Gln Leu Leu Ile Ile Pro Gln His Tyr Ala Val Leu Lys Lys Ala
                405                 410                 415

Glu Arg Glu Gly Cys Gln Tyr Ile Ala Ile Lys Thr Asn Ala Asn Ala
            420                 425                 430

Phe Val Ser His Leu Ala Gly Lys Asn Ser Val Phe Arg Ala Leu Pro
        435                 440                 445

Val Asp Val Val Ala Asn Ala Tyr Arg Ile Ser Arg Glu Gln Ala Arg
    450                 455                 460

Ser Leu Lys Asn Asn Arg Gly Glu Glu His Gly Ala Phe Thr Pro Arg
465                 470                 475                 480

Phe Gln Gln Gln Tyr Tyr Pro Gly Leu Ser Asn Glu Ser Glu Ser Glu
                485                 490                 495

Thr Ser Glu

<210>  5
<211>  500
<212>  PRT
<213>  Oryza sativa

<400>  5

Met Ala Thr Ile Ala Phe Ser Arg Leu Ser Ile Tyr Phe Cys Val Leu
1               5                   10                  15

Leu Leu Cys His Gly Ser Met Ala Gln Leu Phe Gly Pro Asn Val Asn
            20                  25                  30

Pro Trp His Asn Pro Arg Gln Gly Gly Phe Arg Glu Cys Arg Phe Asp
        35                  40                  45

Arg Leu Gln Ala Phe Glu Pro Leu Arg Arg Val Arg Ser Glu Ala Gly
    50                  55                  60

Val Thr Glu Tyr Phe Asp Glu Lys Asn Glu Gln Phe Gln Cys Thr Gly
65                  70                  75                  80

Thr Phe Val Ile Arg Arg Val Ile Glu Pro Gln Gly Leu Leu Val Pro
                85                  90                  95

Arg Tyr Ser Asn Thr Pro Gly Met Val Tyr Ile Ile Gln Gly Arg Gly
            100                 105                 110

Ser Met Gly Leu Thr Phe Pro Gly Cys Pro Ala Thr Tyr Gln Gln Gln
        115                 120                 125

Phe Gln Gln Phe Leu Pro Glu Gly Gln Ser Gln Ser Gln Lys Phe Arg
    130                 135                 140

Asp Glu His Gln Lys Ile His Gln Phe Arg Gln Gly Asp Ile Val Ala
145                 150                 155                 160

Leu Pro Ala Gly Val Ala His Trp Phe Tyr Asn Glu Gly Asp Ala Pro
                165                 170                 175

Val Val Ala Leu Tyr Val Phe Asp Leu Asn Asn Asn Ala Asn Gln Leu
            180                 185                 190

Glu Pro Arg Gln Lys Glu Phe Leu Leu Ala Gly Asn Asn Asn Arg Glu
        195                 200                 205

Gln Gln Met Tyr Gly Arg Ser Ile Glu Gln His Ser Gly Gln Asn Ile
    210                 215                 220

Phe Ser Gly Phe Asn Asn Glu Leu Leu Ser Glu Ala Leu Gly Val Asn
225                 230                 235                 240

Ala Leu Val Ala Lys Arg Leu Gln Gly Gln Asn Asp Gln Arg Gly Glu
                245                 250                 255

Ile Ile Arg Val Lys Asn Gly Leu Lys Leu Leu Arg Pro Ala Phe Ala
            260                 265                 270

Gln Gln Gln Glu Gln Ala Gln Gln Gln Glu Gln Ala Gln Ala Gln Tyr
        275                 280                 285

Gln Val Gln Tyr Ser Glu Glu Gln Gln Pro Ser Thr Arg Cys Asn Gly
    290                 295                 300

Leu Asp Glu Asn Phe Cys Thr Ile Lys Ala Arg Leu Asn Ile Glu Asn
305                 310                 315                 320

Pro Ser His Ala Asp Thr Tyr Asn Pro Arg Ala Gly Arg Ile Thr Arg
                325                 330                 335

Leu Asn Ser Gln Lys Phe Pro Ile Leu Asn Leu Val Gln Leu Ser Ala
            340                 345                 350

Thr Arg Val Asn Leu Tyr Gln Asn Ala Ile Leu Ser Pro Phe Trp Asn
        355                 360                 365

Val Asn Ala His Ser Leu Val Tyr Ile Val Gln Gly His Ala Arg Val
    370                 375                 380

Gln Val Val Ser Asn Leu Gly Lys Thr Val Phe Asn Gly Val Leu Arg
385                 390                 395                 400

Pro Gly Gln Leu Leu Ile Ile Pro Gln His Tyr Val Val Leu Lys Lys
                405                 410                 415

Ala Glu His Glu Gly Cys Gln Tyr Ile Ser Phe Lys Thr Asn Ala Asn
            420                 425                 430

Ser Met Val Ser His Leu Ala Gly Lys Asn Ser Ile Phe Arg Ala Met
        435                 440                 445

Pro Val Asp Val Ile Ala Asn Ala Tyr Arg Ile Ser Arg Glu Gln Ala
    450                 455                 460

Arg Ser Leu Lys Asn Asn Arg Gly Glu Glu Leu Gly Ala Phe Thr Pro
465                 470                 475                 480

Arg Tyr Gln Gln Gln Thr Tyr Pro Gly Phe Ser Asn Glu Ser Glu Asn
                485                 490                 495

Glu Ala Leu Glu
            500

<210>  6
<211>  499
<212>  PRT
<213>  Oryza sativa

<400>  6

Met Ala Ser Ile Asn Arg Pro Ile Val Phe Phe Thr Val Cys Leu Phe
1               5                   10                  15

Leu Leu Cys Asn Gly Ser Leu Ala Gln Gln Leu Leu Gly Gln Ser Thr
            20                  25                  30

Ser Gln Trp Gln Ser Ser Arg Arg Gly Ser Pro Arg Glu Cys Arg Phe
        35                  40                  45

Asp Arg Leu Gln Ala Phe Glu Pro Ile Arg Ser Val Arg Ser Gln Ala
    50                  55                  60

Gly Thr Thr Glu Phe Phe Asp Val Ser Asn Glu Gln Phe Gln Cys Thr
65                  70                  75                  80

Gly Val Ser Val Val Arg Arg Val Ile Glu Pro Arg Gly Leu Leu Leu
                85                  90                  95

Pro His Tyr Thr Asn Gly Ala Ser Leu Val Tyr Ile Ile Gln Gly Arg
            100                 105                 110

Gly Ile Thr Gly Pro Thr Phe Pro Gly Cys Pro Glu Ser Tyr Gln Gln
        115                 120                 125

Gln Phe Gln Gln Ser Gly Gln Ala Gln Leu Thr Glu Ser Gln Ser Gln
    130                 135                 140

Ser Gln Lys Phe Lys Asp Glu His Gln Lys Ile His Arg Phe Arg Gln
145                 150                 155                 160

Gly Asp Val Ile Ala Leu Pro Ala Gly Val Ala His Trp Cys Tyr Asn
                165                 170                 175

Asp Gly Glu Val Pro Val Val Ala Ile Tyr Val Thr Asp Leu Asn Asn
            180                 185                 190

Gly Ala Asn Gln Leu Asp Pro Arg Gln Arg Asp Phe Leu Leu Ala Gly
        195                 200                 205

Asn Lys Arg Asn Pro Gln Ala Tyr Arg Arg Glu Val Glu Glu Arg Ser
    210                 215                 220

Gln Asn Ile Phe Ser Gly Phe Ser Thr Glu Leu Leu Ser Glu Ala Leu
225                 230                 235                 240

Gly Val Ser Ser Gln Val Ala Arg Gln Leu Gln Cys Gln Asn Asp Gln
                245                 250                 255

Arg Gly Glu Ile Val Arg Val Glu His Gly Leu Ser Leu Leu Gln Pro
            260                 265                 270

Tyr Ala Ser Leu Gln Glu Gln Glu Gln Gly Gln Val Gln Ser Arg Glu
        275                 280                 285

Arg Tyr Gln Glu Gly Gln Tyr Gln Gln Ser Gln Tyr Gly Ser Gly Cys
    290                 295                 300

Ser Asn Gly Leu Asp Glu Thr Phe Cys Thr Leu Arg Val Arg Gln Asn
305                 310                 315                 320

Ile Asp Asn Pro Asn Arg Ala Asp Thr Tyr Asn Pro Arg Ala Gly Arg
                325                 330                 335

Val Thr Asn Leu Asn Thr Gln Asn Phe Pro Ile Leu Ser Leu Val Gln
            340                 345                 350

Met Ser Ala Val Lys Val Asn Leu Tyr Gln Asn Ala Leu Leu Ser Pro
        355                 360                 365

Phe Trp Asn Ile Asn Ala His Ser Val Val Tyr Ile Thr Gln Gly Arg
    370                 375                 380

Ala Arg Val Gln Val Val Asn Asn Asn Gly Lys Thr Val Phe Asn Gly
385                 390                 395                 400

Glu Leu Arg Arg Gly Gln Leu Leu Ile Ile Pro Gln His Tyr Ala Val
                405                 410                 415

Val Lys Lys Ala Gln Arg Glu Gly Cys Ala Tyr Ile Ala Phe Lys Thr
            420                 425                 430

Asn Pro Asn Ser Met Val Ser His Ile Ala Gly Lys Ser Ser Ile Phe
        435                 440                 445

Arg Ala Leu Pro Asn Asp Val Leu Ala Asn Ala Tyr Arg Ile Ser Arg
    450                 455                 460

Glu Glu Ala Gln Arg Leu Lys His Asn Arg Gly Asp Glu Phe Gly Ala
465                 470                 475                 480

Phe Thr Pro Ile Gln Tyr Lys Ser Tyr Gln Asp Val Tyr Asn Ala Ala
                485                 490                 495

Glu Ser Ser

<210>  7
<211>  9
<212>  PRT
<213>  Artificial Sequence

<220>
<223>  FRAGMENT OF SEQ ID 1

<400>  7

Gly Ser Leu Leu Leu Pro His Tyr Asn
1               5


<210>  8
<211>  10
<212>  PRT
<213>  Artificial Sequence

<220>
<223>  FRAGMENT OF SEQ ID 1

<400>  8

Gly Ser Leu Leu Leu Pro His Tyr Asn Ser
1               5                   10


<210>  9
<211>  10
<212>  PRT
<213>  Artificial Sequence

<220>
<223>  FRAGEMENT OF SEQ ID 1

<400>  9

Ser Ser Asn Leu Asp Leu Leu Gly Phe Gly
1               5                   10


<210>  10
<211>  8
<212>  PRT
<213>  Artificial Sequence

<220>
<223>  FRAGMENT OF SEQ ID 2

<400>  10

Glu Leu Leu Gly Leu Lys Asn Glu
1               5


<210>  11
<211>  9
<212>  PRT
<213>  Artificial Sequence

<220>
<223>  FRAGMENT OF SEQ ID 2

<400>  11

Ser Asn Leu Asn Leu Leu Gly Phe Gly
1               5


<210>  12
<211>  8
<212>  PRT
<213>  Artificial Sequence

<220>
<223>  FRAGMENT OF SEQ ID 2

<400>  12

Ala Ser Ser Asn Leu Asn Leu Leu
1               5


<210>  13
<211>  9
<212>  PRT
<213>  Artificial Sequence

<220>
<223>  FRAGMENT OF SEQ ID 2

<400>  13

Tyr Pro Gln Leu Gln Asp Leu Asp Leu
1               5


<210>  14
<211>  10
<212>  PRT
<213>  Artificial Sequence

<220>
<223>  FRAGMENT OF SEQ ID 2

<400>  14

Ser Ser Asn Leu Asn Leu Leu Gly Phe Gly
1               5                   10


<210>  15
<211>  9
<212>  PRT
<213>  Artificial Sequence

<220>
<223>  FRAGMENT OF SEQ ID 2

<400>  15

Ala Ser Ser Asn Leu Asn Leu Leu Gly

1               5


<210>  16
<211>  10
<212>  PRT
<213>  Artificial Sequence

<220>
<223>  FRAGMENT OF SEQ ID 2

<400>  16

Leu Leu Gly Leu Lys Asn Glu Gln Gln Glu
1               5                   10


<210>  17
<211>  10
<212>  PRT
<213>  Artificial Sequence

<220>
<223>  FRAGMENT OF SEQ ID 2

<400>  17

Leu Val Val Leu Ser Gly Lys Ala Ile Leu
1               5                   10


<210>  18
<211>  8
<212>  PRT
<213>  Artificial Sequence

<220>
<223>  FRAGMENT OF SEQ ID 2

<400>  18

Leu Asn Leu Leu Gly Phe Gly Ile
1               5


<210>  19
<211>  10
<212>  PRT
<213>  Artificial Sequence

<220>
<223>  FRAGMENT OF SEQ ID 2

<400>  19

Ala Ser Ser Asn Leu Asn Leu Leu Gly Phe
1               5                   10


<210>  20
<211>  8
<212>  PRT
<213>  Artificial Sequence

<220>
<223>  FRAGMENT OF SEQ ID 3

<400>  20

Leu Gln Asn Tyr Arg Leu Leu Glu
1               5


<210>  21
<211>  12
<212>  PRT
<213>  Artificial Sequence

<220>
<223>  FRAGMENT OF SEQ ID 3

<400>  21

Leu Leu Ser Gly Thr Gln Asn Gln Pro Ser Leu Leu
1               5                   10


<210>  22
<211>  9
<212>  PRT
<213>  Artificial Sequence

<220>
<223>  FRAGMENT OF SEQ ID 3

<400>  22

Leu Leu Leu Pro Asn Tyr Asn Ser Arg
1               5


<210>  23
<211>  9
<212>  PRT
<213>  Artificial Sequence

<220>
<223>  FRAGMENT OF SEQ ID 3

<400>  23

Asn Leu Gln Asn Tyr Arg Leu Leu Glu
1               5


<210>  24
<211>  10
<212>  PRT
<213>  Artificial Sequence

<220>
<223>  FRAGMENT OF SEQ ID 3

<400>  24

Gly Ser Leu Leu Leu Pro Asn Tyr Asn Ser

1                   5                       10


<210>  25
<211>  8
<212>  PRT
<213>  Artificial Sequence

<220>
<223>  FRAGMENT OF SEQ ID 3

<400>  25

Asn Leu Gln Asn Tyr Arg Leu Leu
1               5


<210>  26
<211>  10
<212>  PRT
<213>  Artificial Sequence

<220>
<223>  FRAGMENT OF SEQ ID 4

<400>  26

Asn Gly Leu Gln Leu Leu Lys Pro Thr Leu
1               5                   10


<210>  27
<211>  9
<212>  PRT
<213>  Artificial Sequence

<220>
<223>  FRAGMENT OF SEQ ID 4

<400>  27

Gly Leu Gln Leu Leu Lys Pro Thr Leu
1               5


<210>  28
<211>  9
<212>  PRT
<213>  Artificial Sequence

<220>
<223>  FRAGMENT OF SEQ ID 4

<400>  28

Gly Val Leu Arg Pro Gly Gln Leu Leu
1               5


<210>  29
<211>  10
<212>  PRT
<213>  Artificial Sequence

<220>
<223>  FRAGMENT OF SEQ ID 4

<400>  29

Asp Gly Val Leu Arg Pro Gly Gln Leu Leu
1               5                   10


<210>  30
<211>  13
<212>  PRT
<213>  Artificial Sequence

<220>
<223>  FRAGMENT OF SEQ ID 4

<400>  30

Leu Gln Leu Leu Lys Pro Thr Leu Thr Gln Gln Gln Glu
1               5                   10


<210>  31
<211>  18
<212>  PRT
<213>  Artificial Sequence

<220>
<223>  FRAGMENT OF SEQ ID 5

<400>  31

Leu Asn Ser Gln Lys Phe Pro Ile Leu Asn Leu Val Gln Leu Ser Ala
1               5                   10                  15


Thr Arg




<210>  32
<211>  10
<212>  PRT
<213>  Artificial Sequence

<220>
<223>  FRAGMENT OF SEQ ID 5

<400>  32

Leu Ser Glu Ala Leu Gly Val Asn Ala Leu
1               5                   10


<210>  33
<211>  10
<212>  PRT
<213>  Artificial Sequence

<220>
<223>  FRAGMENT OF SEQ ID 6

<400>  33

His Gly Leu Ser Leu Leu Gln Pro Tyr Ala
1               5                   10

<210>  34
<211>  12
<212>  PRT
<213>  Artificial Sequence

<220>
<223>  FRAGMENT OF SEQ ID 6

<400>  34

His Gly Leu Ser Leu Leu Gln Pro Tyr Ala Ser Leu
1               5                   10

<210>  35
<211>  9
<212>  PRT
<213>  Artificial Sequence

<220>
<223>  FRAGMENT OF SEQ ID 6

<400>  35

His Gly Leu Ser Leu Leu Gln Pro Tyr
1               5

<210>  36
<211>  446
<212>  PRT
<213>  Cicer arietinum

<400>  36

Met Ala Ile Lys Ala Arg Phe Pro Leu Leu Val Leu Leu Gly Ile Val
1               5                   10                  15

Phe Leu Ala Ser Val Cys Ala Lys Ser Asp Lys Glu Asn Pro Phe Phe
            20                  25                  30

Phe Lys Ser Asn Asn Cys Gln Thr Leu Phe Glu Asn Glu Asn Gly His
        35                  40                  45

Val Arg Leu Leu Gln Arg Phe Asp Lys Arg Ser Gln Leu Phe Glu Asn
    50                  55                  60

Leu Gln Asn Tyr Arg Leu Met Glu Tyr Asn Ser Lys Pro His Thr Leu
65                  70                  75                  80

Phe Leu Pro Gln His Asn Asp Ala Asp Phe Ile Leu Val Val Leu Arg

85                  90                  95

Gly Arg Ala Ile Leu Thr Val Leu Asn Pro Asn Asp Arg Asn Thr Phe
            100                 105                 110

Lys Leu Glu Arg Gly Asp Thr Ile Lys Leu Pro Ala Gly Thr Ile Ala
        115                 120                 125

Tyr Leu Ala Asn Arg Asp Asp Asn Glu Asp Leu Arg Val Leu Asp Leu
    130                 135                 140

Ala Ile Pro Val Asn Arg Pro Gly Gln Phe Gln Ser Phe Ser Leu Ser
145                 150                 155                 160

Gly Asn Glu Asn Gln Gln Ser Tyr Phe Gln Gly Phe Ser Lys Lys Ile
                165                 170                 175

Leu Glu Ala Ser Phe Asn Ser Asp Tyr Glu Glu Ile Glu Arg Val Leu
            180                 185                 190

Leu Glu Glu Gln Glu Gln Lys Pro Glu Gln Arg Arg Gly His Lys Gly
        195                 200                 205

Arg Gln Gln Ser Gln Glu Thr Asp Val Ile Val Lys Ile Ser Arg Glu
    210                 215                 220

Gln Ile Glu Glu Leu Ser Lys Asn Ala Lys Ser Asn Cys Lys Lys Ser
225                 230                 235                 240

Val Ser Ser Glu Ser Glu Pro Phe Asn Leu Arg Ser Arg Ser Pro Ile
                245                 250                 255

Tyr Ser Asn Arg Phe Gly Asn Phe Phe Glu Ile Thr Pro Glu Lys Asn
            260                 265                 270

Pro Gln Leu Lys Asp Leu Asp Ile Phe Val Asn Ser Val Glu Ile Lys
        275                 280                 285

Glu Gly Ser Leu Leu Leu Pro His Phe Asn Ser Arg Ala Thr Val Ile
    290                 295                 300

Leu Val Val Asn Glu Gly Lys Gly Glu Val Glu Leu Val Gly Leu Arg
305                 310                 315                 320

Asn Glu Asn Glu Gln Glu Asn Lys Lys Glu Asp Glu Glu Glu Glu Glu
                325                 330                 335

Asp Arg Asn Val Gln Val Gln Arg Phe Gln Ser Lys Leu Ser Ser Gly
            340                 345                 350

Asp Val Val Val Ile Pro Ala Ser His Pro Phe Ser Ile Asn Ala Ser
        355                 360                 365

Ser Asp Leu Phe Leu Leu Gly Phe Gly Ile Asn Ala Gln Asn Asn Gln
    370                 375                 380

Arg Asn Phe Leu Ala Gly Glu Glu Asp Asn Val Ile Ser Gln Ile Gln
385                 390                 395                 400

Arg Pro Val Lys Glu Val Ala Phe Pro Gly Ser Ala Glu Glu Val Asp
                405                 410                 415

Arg Leu Leu Lys Asn Gln Arg Gln Ser His Phe Ala Asn Ala Gln Pro
            420                 425                 430

Gln Gln Lys Arg Lys Gly Ser Gln Arg Ile Arg Ser Pro Phe
        435                 440                 445

<210>  37
<211>  418
<212>  PRT
<213>  Lens culinaris

<400>  37

Ser Arg Ser Asp Gln Glu Asn Pro Phe Ile Phe Lys Ser Asn Arg Phe
1               5                   10                  15

Gln Thr Ile Tyr Glu Asn Glu Asn Gly His Ile Arg Leu Leu Gln Arg
            20                  25                  30

Phe Asp Lys Arg Ser Lys Ile Phe Glu Asn Leu Gln Asn Tyr Arg Leu
        35                  40                  45

Leu Glu Tyr Lys Ser Lys Pro His Thr Ile Phe Leu Pro Gln Phe Thr
    50                  55                  60

Asp Ala Asp Phe Ile Leu Val Val Leu Ser Gly Lys Ala Ile Leu Thr
65                  70                  75                  80

Val Leu Asn Ser Asn Asp Arg Asn Ser Phe Asn Leu Glu Arg Gly Asp
                85                  90                  95

Thr Ile Lys Leu Pro Ala Gly Thr Ile Ala Tyr Leu Ala Asn Arg Asp
            100                 105                 110

Asp Asn Glu Asp Leu Arg Val Leu Asp Leu Ala Ile Pro Val Asn Arg
        115                 120                 125

Pro Gly Gln Leu Gln Ser Phe Leu Leu Ser Gly Thr Gln Asn Gln Pro
    130                 135                 140

Ser Phe Leu Ser Gly Phe Ser Lys Asn Ile Leu Glu Ala Ala Phe Asn
145                 150                 155                 160

Thr Glu Tyr Glu Glu Ile Glu Lys Val Leu Leu Glu Glu Gln Glu Gln
                165                 170                 175

Lys Ser Gln His Arg Arg Ser Leu Arg Asp Lys Arg Gln Glu Ile Thr
            180                 185                 190

Asn Glu Asp Val Ile Val Lys Val Ser Arg Glu Gln Ile Glu Glu Leu
        195                 200                 205

Ser Lys Asn Ala Lys Ser Ser Ser Lys Lys Ser Val Ser Ser Glu Ser
    210                 215                 220

Glu Pro Phe Asn Leu Arg Ser Arg Asn Pro Ile Tyr Ser Asn Lys Phe
225                 230                 235                 240

Gly Lys Phe Phe Glu Ile Thr Pro Glu Lys Asn Pro Gln Leu Gln Asp
                245                 250                 255

Leu Asp Ile Phe Val Asn Ser Val Glu Ile Lys Glu Gly Ser Leu Leu
            260                 265                 270

Leu Pro Asn Tyr Asn Ser Arg Ala Ile Val Ile Val Thr Val Asn Glu
        275                 280                 285

Gly Lys Gly Asp Phe Glu Leu Val Gly Gln Arg Asn Glu Asn Gln Gln
    290                 295                 300

Glu Gln Arg Glu Glu Asn Asp Glu Glu Glu Gly Gln Glu Glu Glu Thr
305                 310                 315                 320

Thr Lys Gln Val Gln Arg Tyr Arg Ala Arg Leu Ser Pro Gly Asp Val
                325                 330                 335

Leu Val Ile Pro Ala Gly His Pro Val Ala Ile Asn Ala Ser Ser Asp
            340                 345                 350

Leu Asn Leu Ile Gly Phe Gly Ile Asn Ala Lys Asn Asn Gln Arg Asn
        355                 360                 365

Phe Leu Ala Gly Glu Glu Asp Asn Val Ile Ser Gln Ile Gln Arg Pro
    370                 375                 380

Val Lys Glu Leu Ala Phe Pro Gly Ser Ser Arg Glu Val Asp Arg Leu
385                 390                 395                 400

Leu Thr Asn Gln Lys Gln Ser His Phe Ala Asn Ala Gln Pro Leu Gln
                405                 410                 415

Ile Glu

<210>  38
<211>  526
<212>  PRT
<213>  Pisum abyssinicum

<400>  38

Met Ala Thr Thr Val Glu Ser Arg Phe Pro Leu Leu Leu Phe Pro Gly
1               5                   10                  15

Ile Ile Phe Leu Ala Ser Val Cys Val Thr Tyr Ala Asn Tyr Asp Glu
            20                  25                  30

Gly Ser Glu Thr Arg Val Pro Gly Gln Arg Glu Arg Gly Arg Gln Glu
        35                  40                  45

Gly Glu Lys Glu Glu Lys Arg His Gly Glu Trp Arg Pro Ser Tyr Glu
    50                  55                  60

Lys Glu Glu Asp Glu Glu Glu Lys Gln Lys Tyr Arg Tyr Gln Arg Glu
65                  70                  75                  80

Lys Glu Asp Glu Glu Glu Lys Gln Lys Tyr Arg Tyr Gln Arg Glu Lys
                85                  90                  95

Lys Glu Glu Lys Glu Val Gln Pro Gly Arg Glu Arg Trp Glu Arg Glu
            100                 105                 110

Glu Asp Glu Glu Gln Val Asp Glu Glu Trp Arg Gly Ser Gln Arg Arg
        115                 120                 125

Gln Asp Pro Glu Glu Arg Ala Arg Leu Arg His Arg Glu Glu Arg Thr
    130                 135                 140

Lys Arg Asp Arg Arg His Lys Arg Glu Gly Glu Glu Glu Glu Arg Ser
145                 150                 155                 160

Ser Glu Ser Gln Glu Gln Arg Asn Pro Phe Leu Phe Lys Ser Asn Lys
                165                 170                 175

Phe Leu Thr Leu Phe Glu Asn Glu Asn Gly His Ile Arg Arg Leu Gln
            180                 185                 190

Arg Phe Asp Lys Arg Ser Asp Leu Phe Glu Asn Leu Gln Asn Tyr Arg
        195                 200                 205

Leu Val Glu Tyr Arg Ala Lys Pro His Thr Ile Phe Leu Pro Gln His
    210                 215                 220

Ile Asp Ala Asp Leu Ile Leu Val Val Leu Asn Gly Lys Ala Ile Leu
225                 230                 235                 240

Thr Val Leu Ser Pro Asn Asp Arg Asn Ser Tyr Asn Leu Glu Arg Gly
                245                 250                 255

Asp Thr Ile Lys Ile Pro Ala Gly Thr Thr Ser Tyr Leu Val Asn Gln
            260                 265                 270

Asp Asp Glu Glu Asp Leu Arg Val Val Asp Phe Val Ile Pro Val Asn
        275                 280                 285

Arg Pro Gly Lys Phe Glu Ala Phe Gly Leu Ser Glu Asn Lys Asn Gln
    290                 295                 300

Tyr Leu Arg Gly Phe Ser Lys Asn Ile Leu Glu Ala Ser Leu Asn Thr
305                 310                 315                 320

Lys Tyr Glu Thr Ile Glu Lys Val Leu Leu Glu Glu Gln Glu Lys Lys
                325                 330                 335

Pro Gln Gln Leu Arg Asp Arg Lys Arg Arg Gln Gln Gly Gly Glu Arg
            340                 345                 350

Asp Ala Ile Ile Lys Val Ser Arg Glu Gln Ile Glu Glu Leu Arg Lys
        355                 360                 365

Leu Ala Lys Ser Ser Ser Lys Lys Ser Leu Pro Ser Glu Phe Glu Pro
    370                 375                 380

Phe Asn Leu Arg Ser His Lys Pro Glu Tyr Ser Asn Lys Phe Gly Lys
385                 390                 395                 400

Leu Phe Glu Ile Thr Pro Glu Lys Lys Tyr Pro Gln Leu Gln Asp Leu
                405                 410                 415

Asp Ile Leu Val Ser Cys Val Glu Ile Asn Lys Gly Ala Leu Met Leu
            420                 425                 430

Pro His Tyr Asn Ser Arg Ala Ile Val Val Leu Leu Val Asn Glu Gly
        435                 440                 445

Lys Gly Asn Leu Glu Leu Leu Gly Leu Lys Asn Glu Gln Gln Glu Arg
    450                 455                 460

Glu Asp Arg Lys Glu Arg Asn Asn Glu Val Gln Arg Tyr Glu Ala Arg
465                 470                 475                 480

Leu Ser Pro Gly Asp Val Val Ile Ile Pro Ala Gly His Pro Val Ala
                485                 490                 495

Ile Ser Ala Ser Ser Asn Leu Asn Leu Leu Gly Phe Gly Thr Asn Ala
            500                 505                 510

Glu Asn Asn Gln Arg Asn Phe Leu Ser Gly Ser Asp Asp Asn
        515                 520                 525

<210>  39
<211>  541
<212>  PRT
<213>  Lathyrus annuus

<400>  39

Met Ala Thr Thr Ile Lys Ser Arg Phe Pro Leu Leu Leu Leu Leu Gly
1               5                   10                  15

Ile Ile Phe Leu Ala Ser Val Cys Val Thr Trp Ala Asn Tyr Asp Glu
            20                  25                  30

Gly Ser Glu Pro Arg Val Pro Gly Gln Arg Glu Arg Gly Arg Gln Glu
        35                  40                  45

Gly Glu Lys Glu Glu Lys Arg His Gly Glu Trp Arg Pro Ser Tyr Glu
    50                  55                  60

Glu Glu Tyr Asp Glu Gly Leu Glu Pro Lys Val Pro Gly Lys Arg Glu
65                  70                  75                  80

Arg Gly Arg Gln Glu Gly Glu Lys Glu Glu Lys Arg His Glu Glu Trp
                85                  90                  95

Arg Pro Ser Tyr Glu Lys Glu Glu Asp Glu Glu Glu Lys Gln Lys Tyr
            100                 105                 110

Asn Tyr Gln Arg Glu Lys Lys Glu His Lys Glu Val Gln Pro Gly Arg
        115                 120                 125

Glu Arg Trp Glu Arg Lys Gln Asp Glu Lys Gln Val Glu Glu Asp Glu
    130                 135                 140

Glu Pro Gly Glu Glu Gln Trp Arg Gly Ser Lys Arg His Glu Asp Pro
145                 150                 155                 160

Glu Glu Arg Ala Arg Leu Arg His Arg Glu Glu Lys Thr Lys Ser Tyr
                165                 170                 175

Val Glu Asp Asn Glu Glu Thr Ser Ser Lys Glu Gly Arg Asn Pro Phe
            180                 185                 190

Leu Phe Lys Ser Asn Lys Phe Leu Thr Leu Phe Glu Asn Glu Asn Gly
        195                 200                 205

His Ile Arg Arg Leu Gln Arg Phe Asp Glu Arg Ser Asp Ile Phe Glu
    210                 215                 220

Asn Leu Gln Asn Tyr Arg Leu Val Glu Tyr Arg Ala Lys Pro His Thr
225                 230                 235                 240

Met Phe Leu Pro Gln His Ile Asp Ala Asp Leu Ile Leu Val Val Leu
                245                 250                 255

Asn Gly Lys Ala Ile Leu Thr Val Leu Ser Pro Asn Asp Arg Asn Ser
            260                 265                 270

Tyr Asn Leu Glu Arg Gly Asp Thr Val Lys Leu Pro Ala Gly Thr Thr
        275                 280                 285

Ser Tyr Leu Val Asn Gln Asp Asp Glu Glu Asp Leu Arg Val Val Asp
    290                 295                 300

Leu Ala Ile Pro Val Asn Arg Pro Gly Lys Phe Glu Ala Phe Gly Leu
305                 310                 315                 320

Ser Ala Asn Lys Asn Gln Tyr Leu Arg Gly Phe Ser Lys Asn Ile Leu
                325                 330                 335

Glu Ala Ser Leu Asn Thr Lys Tyr Glu Thr Ile Glu Lys Val Leu Leu
            340                 345                 350

Glu Glu Arg Arg Asp Gln Lys Gly Arg Gln Gln Gly Gln Glu Thr Asn

355                 360                 365

Ala Ile Val Lys Val Ser Arg Glu Gln Ile Glu Glu Leu Arg Lys Leu
    370                 375                 380

Ala Lys Ser Ser Ser Lys Lys Ser Leu Leu Ser Glu Ser Glu Pro Leu
385                 390                 395                 400

Asn Leu Arg Ser Gln Asn Pro Lys Tyr Ser Asn Lys Phe Gly Lys Phe
                405                 410                 415

Phe Glu Ile Thr Pro Gln Lys Lys Tyr Pro Gln Leu Gln Asp Leu Asp
            420                 425                 430

Val Ser Ile Ser Cys Val Glu Ile Asn Lys Gly Ala Leu Leu Leu Pro
        435                 440                 445

His Tyr Asn Ser Arg Ser Ile Gly Ile Leu Leu Val Asn Glu Gly Lys
    450                 455                 460

Gly Asn Leu Glu Leu Val Gly Phe Lys Asn Glu Gln Gln Arg Gln Arg
465                 470                 475                 480

Glu Asn Glu Glu Thr Asn Lys Lys Leu Gln Arg Tyr Glu Ala Arg Leu
                485                 490                 495

Ser Ser Gly Asp Val Val Val Ile Pro Glu Gly His Pro Val Ala Ile
            500                 505                 510

Ser Ala Ser Ser Asn Leu Asn Leu Leu Gly Phe Gly Ile Asn Ala Ala
        515                 520                 525

Asn Asn Gln Arg Asn Phe Leu Thr Gly Ser Asp Asp Asn
    530                 535                 540

<210>  40
<211>  589
<212>  PRT
<213>  Vicia villosa

<400>  40

Met Ala Thr Thr Ile Lys Ser Arg Phe Pro Val Leu Leu Leu Leu Gly
1               5                   10                  15

Ile Ile Phe Leu Thr Ser Val Cys Val Thr Tyr Ala Asn Tyr Asp Glu
            20                  25                  30

Gly Arg Glu Pro Ser Val Pro Gly Gln Arg Glu Arg Gly Arg Gln Glu

35                  40                  45

Gly Glu Lys Glu Glu Lys Arg His Gly Glu Trp Arg Pro Ser Glu Glu
    50                  55                  60

Asp Glu Glu Glu Lys Tyr Lys Tyr Glu Glu Gly Arg Val Pro Gly Gln
65                  70                  75                  80

Arg Glu Arg Gly Arg Gln Glu Gly Glu Lys Glu Glu Lys Arg His Gly
                85                  90                  95

Lys Trp Arg Pro Ser Glu Glu Glu Asp Glu Glu Glu Lys Tyr Arg Tyr
            100                 105                 110

Glu Glu Gly Ser Glu Pro Arg Gly Pro Gly Gln Arg Glu Thr Gly Arg
        115                 120                 125

Gln Glu Gly Glu Lys Glu Lys Gln Arg Pro Glu Arg Glu Pro Ser Tyr
    130                 135                 140

Glu Lys Glu Glu Asp Glu Glu Glu Lys Gln Lys Tyr Gln Tyr His Arg
145                 150                 155                 160

Glu Lys Lys Glu Gln Arg Glu Val Arg Pro Gly Arg Glu Arg Phe Glu
                165                 170                 175

Arg His Glu Asp Glu Glu Gln Trp Arg Gly Ile Gln Arg His Glu Asp
            180                 185                 190

Pro Glu Glu Arg Ala Arg Glu Arg Tyr Arg Ala Glu Ile Ala Lys Arg
        195                 200                 205

Gln Val Glu Glu Glu Arg Glu Glu Arg Asp Ile Pro His Glu Arg Glu
    210                 215                 220

Gln Arg Asn Pro Phe Leu Phe Lys Ser Asn Lys Phe Gln Thr Leu Phe
225                 230                 235                 240

Gln Asn Glu Asn Gly Tyr Ile Arg Arg Leu Gln Arg Phe Asp Lys Arg
                245                 250                 255

Ser Asp Leu Phe Glu Asn Leu Gln Asn Tyr Arg Leu Val Glu Tyr Arg
            260                 265                 270

Ala Lys Pro His Thr Ile Phe Leu Pro Gln His Ile Asp Ala Asp Leu
        275                 280                 285

Ile Ile Val Val Leu Ser Gly Arg Ala Ile Leu Thr Val Leu Ser Pro
    290                 295                 300

Asp Asp Arg Asn Ser Tyr Asn Leu Glu Arg Gly Asp Thr Ile Lys Leu
305                 310                 315                 320

Pro Ala Gly Thr Thr Ser Tyr Leu Val Asn Gln Asp Asp Glu Glu Asp
                325                 330                 335

Leu Arg Val Val Asp Leu Ala Ile Pro Val Asn Arg Pro Gly Lys Val
            340                 345                 350

Glu Ser Phe Leu Leu Ser Gly Asn Lys Asn Gln Tyr Leu Arg Gly Phe
        355                 360                 365

Ser Lys Asn Ile Leu Glu Ala Ser Phe Asn Thr Asn Tyr Glu Thr Ile
    370                 375                 380

Glu Arg Val Leu Leu Glu Glu Gln Asp Lys Glu Ser Gln Gln Ser Ile
385                 390                 395                 400

Gly Gln Lys Arg Arg Ser Gln Arg Gln Glu Thr Asn Ala Leu Val Lys
                405                 410                 415

Val Ser Arg Glu Gln Leu Glu Asp Leu Lys Arg Leu Ala Lys Ser Ser
            420                 425                 430

Ser Gln Glu Gly Leu Ser Ser Gln Phe Glu Pro Ile Asn Leu Arg Ser
        435                 440                 445

Gln Asn Pro Lys Tyr Ser Asn Lys Phe Gly Lys Val Phe Glu Ile Thr
    450                 455                 460

Pro Glu Lys Lys Tyr Pro Gln Leu Gln Asp Leu Asp Leu Phe Val Ser
465                 470                 475                 480

Ser Val Asp Ile Lys Glu Gly Ala Leu Met Leu Pro His Tyr Asn Ser
                485                 490                 495

Arg Ala Ile Val Val Leu Leu Val Asn Glu Gly Arg Gly Asn Leu Glu
            500                 505                 510

Leu Val Gly Leu Lys Asn Glu Gln Gln Glu Gln Arg Glu Lys Glu Asp
        515                 520                 525

Glu Gln Gln Glu Arg Asn Asn Gln Val Gln Arg Tyr Glu Ala Arg Leu
    530                 535                 540

Ser Pro Gly Asp Val Val Ile Ile Pro Ala Gly His Pro Val Ala Val
545                 550                 555                 560

Arg Ala Ser Ser Asp Leu Asn Leu Leu Ala Phe Gly Ile Asn Ala Glu
                565                 570                 575

Asn Asn Gln Arg Asn Phe Leu Ala Gly Ser Asp Asp Asn
            580                 585

<210>  41
<211>  415
<212>  PRT
<213>  Lens culinaris

<400>  41

Ser Arg Ser Asp Gln Glu Asn Pro Phe Ile Phe Lys Ser Asn Arg Phe
1               5                   10                  15

Gln Thr Ile Tyr Glu Asn Glu Asn Gly His Ile Arg Leu Leu Gln Lys
            20                  25                  30

Phe Asp Lys Arg Ser Lys Ile Phe Glu Asn Leu Gln Asn Tyr Arg Leu
        35                  40                  45

Leu Glu Tyr Lys Ser Lys Pro His Thr Leu Phe Leu Pro Gln Tyr Thr
    50                  55                  60

Asp Ala Asp Phe Ile Leu Val Val Leu Ser Gly Lys Ala Val Leu Thr
65                  70                  75                  80

Val Leu Asn Ser Asn Asp Arg Asn Ser Phe Asn Leu Glu Arg Gly Asp
                85                  90                  95

Thr Ile Lys Leu Pro Ala Gly Thr Ile Ala Tyr Leu Ala Asn Arg Asp
            100                 105                 110

Asp Asn Glu Asp Leu Arg Val Leu Asp Leu Ala Ile Pro Val Asn Asn
        115                 120                 125

Pro Gly Gln Leu Glu Ser Phe Leu Leu Ser Gly Thr Gln Asn Gln Pro
    130                 135                 140

Ser Phe Leu Ser Gly Phe Asn Lys Ser Ile Leu Glu Ala Ala Phe Asn
145                 150                 155                 160

Thr Asp Tyr Glu Glu Ile Glu Lys Val Leu Leu Glu Asp Gln Glu Gln
                165                 170                 175

Glu Pro Gln His Arg Arg Ser Leu Arg Asp Arg Arg Gln Glu Ile Asn
        180                 185                 190

Lys Glu Asn Val Ile Val Lys Val Ser Arg Glu Gln Ile Lys Glu Leu
    195                 200                 205

Ser Lys Asn Ala Lys Ser Ser Ser Lys Lys Ser Val Ser Ser Glu Ser
    210                 215                 220

Glu Pro Phe Asn Leu Arg Ser Arg Asn Pro Ile Tyr Ser Asn Lys Phe
225                 230                 235                 240

Gly Lys Phe Phe Glu Ile Thr Pro Glu Lys Asn Pro Gln Leu Gln Asp
                245                 250                 255

Leu Asp Ile Phe Val Asn Ser Val Glu Ile Lys Glu Gly Ser Leu Leu
            260                 265                 270

Leu Pro Asn Tyr Asn Ser Arg Ala Ile Val Ile Val Thr Val Asn Glu
        275                 280                 285

Gly Lys Gly Tyr Phe Glu Leu Val Gly Gln Arg Asn Glu Asn Gln Arg
    290                 295                 300

Glu Glu Asn Asp Asp Glu Glu Glu Gln Glu Glu Glu Thr Ser Thr Gln
305                 310                 315                 320

Val Gln Arg Tyr Arg Ala Lys Leu Ser Pro Gly Asp Val Phe Val Val
                325                 330                 335

Pro Ala Gly His Pro Val Ala Ile Asn Ala Ser Ser Asp Leu Asn Leu
            340                 345                 350

Ile Gly Phe Gly Ile Asn Ala Lys Asn Asn Gln Arg Asn Phe Leu Ala
        355                 360                 365

Gly Glu Glu Asp Asn Val Ile Ser Gln Ile Gln Arg Pro Val Lys Glu
    370                 375                 380

Leu Ala Phe Pro Gly Ser Ser Arg Glu Val Asp Arg Leu Leu Thr Asn
385                 390                 395                 400

Gln Lys Gln Ser His Phe Ala Asn Ala Gln Pro Leu Gln Ile Glu
                405                 410                 415

<210>  42
<211>  463

<212>  PRT
<213>  Vicia narbonensis

<400>  42

Met Ala Ala Ile Thr Met Lys Val Ser Phe Pro Leu Leu Met Leu Leu
1               5                   10                  15

Gly Ile Ser Phe Leu Ala Ser Val Cys Val Ser Ser Arg Ser Asp Gln
            20                  25                  30

Glu Asn Pro Phe Ile Phe Lys Ser Asn Lys Phe Gln Thr Leu Phe Glu
        35                  40                  45

Asn Asp Asn Gly His Ile Arg Leu Leu Gln Lys Phe Asp Glu Arg Ser
    50                  55                  60

Lys Ile Leu Glu Asn Leu Gln Asn Tyr Arg Leu Leu Glu Tyr Lys Ser
65                  70                  75                  80

Lys Pro Arg Thr Ile Phe Leu Pro Gln Gln Thr Asn Ala Asp Phe Ile
                85                  90                  95

Leu Val Val Leu Ser Gly Lys Ala Ile Leu Thr Val Leu Lys Pro Asp
            100                 105                 110

Asp Arg Asn Ser Phe Asn Leu Glu Arg Gly Asp Thr Ile Lys Leu Pro
        115                 120                 125

Ala Gly Thr Ile Ala Tyr Leu Val Asn Lys Asp Asp Asn Glu Asp Leu
    130                 135                 140

Arg Val Leu Asp Leu Ala Ile Pro Val Asn Gly Pro Asp Gln Leu Gln
145                 150                 155                 160

Ser Phe Leu Leu Ser Gly Ser Glu Asn Gln Gln Ser Ile Leu Ser Gly
                165                 170                 175

Phe Ser Lys Ser Val Leu Glu Ala Ser Phe Asn Thr Gly Tyr Glu Glu
            180                 185                 190

Ile Glu Lys Val Leu Leu Glu Glu Arg Glu Lys Glu Thr Gln His Arg
        195                 200                 205

Arg Ser Leu Arg Asp Lys Arg Gln His Ser Gln Asp Glu Asp Val Ile
    210                 215                 220

Val Lys Leu Ser Arg Gly Gln Ile Glu Glu Leu Ser Arg Asn Ala Lys
225                 230                 235                 240

Ser Ser Ser Lys Lys Ser Val Ser Ser Glu Ser Glu Pro Phe Asn Leu
                245                 250                 255

Arg Ser Arg Asn Pro Ile Tyr Ser Asn Lys Phe Gly Lys Phe Phe Glu
            260                 265                 270

Ile Thr Pro Glu Lys Asn Pro Gln Leu Gln Asp Leu Asp Val Leu Val
        275                 280                 285

Asn Ser Val Glu Ile Lys Glu Gly Ser Leu Leu Leu Pro His Tyr Asn
    290                 295                 300

Ser Arg Ala Ile Val Ile Val Thr Val Asn Asp Gly Lys Gly Asp Phe
305                 310                 315                 320

Glu Ile Val Gly Gln Arg Asn Glu Asn Arg Gln Gly Gln Arg Lys Glu
                325                 330                 335

Asp Asp Glu Glu Glu Glu Gln Gly Asp Glu Asn Thr Asn Thr Gln Val
            340                 345                 350

Gln Asn Tyr Lys Ala Lys Leu Ser Arg Gly Asp Val Phe Val Ile Pro
        355                 360                 365

Ala Gly His Pro Val Ser Ile Lys Ala Ser Ser Asn Leu Asp Leu Leu
    370                 375                 380

Gly Phe Gly Ile Asn Ala Lys Asn Asn Gln Arg Asn Phe Leu Ala Gly
385                 390                 395                 400

Glu Glu Asp Asn Val Ile Ser Gln Ile Asp Arg Pro Val Lys Glu Leu
                405                 410                 415

Ala Phe Pro Gly Ser Ala Gln Glu Val Asp Arg Leu Leu Glu Asn Gln
            420                 425                 430

Lys Gln Ser His Phe Ala Asn Ala Gln Pro Gln Gln Arg Glu Arg Gly
        435                 440                 445

Ser His Glu Thr Arg Asp His Leu Ser Ser Ile Leu Asp Ala Phe
    450                 455                 460

<210>  43
<211>  463
<212>  PRT
<213>  Medicago truncatula

<400>  43

Met Ala Ile Lys Ala Pro Phe Gln Leu Leu Met Leu Leu Gly Ile Phe
1               5                   10                  15

Phe Leu Ala Ser Val Cys Val Ser Ser Arg Asp Asp Arg His Asp Gln
            20                  25                  30

Glu Asn Pro Phe Phe Phe Asn Ala Asn His Phe Gln Thr Leu Phe Glu
        35                  40                  45

Asn Glu Asn Gly His Ile Arg Leu Leu Gln Arg Phe Asp Lys Arg Ser
    50                  55                  60

Lys Ile Phe Glu Asn Leu Gln Asn Tyr Arg Leu Leu Glu Tyr His Ser
65                  70                  75                  80

Lys Pro His Thr Leu Phe Leu Pro Gln His Asn Asp Ala Asp Phe Ile
                85                  90                  95

Leu Ala Val Leu Ser Gly Lys Ala Ile Leu Thr Val Leu Asn Pro Asp
            100                 105                 110

Asn Arg Asn Ser Phe Asn Leu Glu Arg Gly Asp Thr Ile Lys Leu Pro
        115                 120                 125

Ala Gly Ser Ile Ala Tyr Leu Ala Asn Arg Asp Asp Asn Glu Asp Leu
    130                 135                 140

Arg Val Leu Asp Leu Ala Ile Pro Val Asn Arg Pro Gly Lys Phe Gln
145                 150                 155                 160

Ser Phe Ser Leu Ser Gly Ser Gln Asn Gln Gln Ser Phe Phe Ser Gly
                165                 170                 175

Phe Ser Lys Asn Ile Leu Glu Ala Ala Phe Asn Ala Asn Tyr Glu Glu
            180                 185                 190

Ile Glu Arg Val Leu Ile Glu Glu His Glu Gln Glu Pro Gln His Arg
        195                 200                 205

Arg Gly Leu Arg Lys Asp Arg Arg Gln Gln Ser Gln Asp Ser Asn Val
    210                 215                 220

Ile Val Lys Val Ser Arg Glu Gln Ile Glu Glu Leu Ser Arg His Ala
225                 230                 235                 240

Lys Ser Ser Ser Arg Arg Ser Gly Ser Ser Glu Ser Ala Pro Phe Asn

245                 250                 255

Leu Arg Ser Arg Glu Pro Ile Tyr Ser Asn Glu Phe Gly Asn Phe Phe
            260                 265                 270

Glu Ile Thr Pro Glu Lys Asn Pro Gln Leu Lys Asp Leu Asp Ile Leu
        275                 280                 285

Val Asn Tyr Ala Glu Ile Arg Glu Gly Ser Leu Leu Leu Pro His Phe
    290                 295                 300

Asn Ser Arg Ala Thr Val Ile Val Val Val Asp Glu Gly Lys Gly Glu
305                 310                 315                 320

Phe Glu Leu Val Gly Gln Arg Asn Glu Asn Gln Gln Glu Gln Arg Glu
                325                 330                 335

Glu Asp Glu Gln Gln Glu Glu Glu Arg Ser Gln Gln Val Gln Arg Tyr
            340                 345                 350

Arg Ala Arg Leu Ser Pro Gly Asp Val Tyr Val Ile Pro Ala Gly His
        355                 360                 365

Pro Thr Val Val Ser Ala Ser Ser Asp Leu Ser Leu Leu Gly Phe Gly
    370                 375                 380

Ile Asn Ala Glu Asn Asn Glu Arg Asn Phe Leu Ala Gly Glu Glu Asp
385                 390                 395                 400

Asn Val Ile Ser Gln Ile Glu Arg Pro Val Lys Glu Val Ala Phe Pro
                405                 410                 415

Gly Ser Ala Gln Asp Val Glu Ser Leu Leu Lys Asn Gln Arg Gln Ser
            420                 425                 430

Tyr Phe Ala Asn Ala Gln Pro Gln Gln Arg Glu Arg Glu Glu Gly Arg
        435                 440                 445

Ser Gln Arg Gln Arg Glu Leu Ile Ser Ser Ile Leu Gly Val Phe
    450                 455                 460

<210>  44
<211>  495
<212>  PRT
<213>  Oryza brachyantha

<400>  44

Met Ala Thr Thr Val Phe Ser Arg Phe Ser Thr Tyr Phe Cys Val Leu

1                   5                   10                  15

Leu Leu Cys His Gly Ser Met Ala Gln Leu Phe Asn Pro Ser Thr Asn
            20                  25                  30

Pro Trp His Asn Pro Arg Gln Gly Ser Ser Arg Glu Cys Arg Phe Asp
        35                  40                  45

Arg Leu Gln Pro Phe Glu Pro Leu Arg Lys Val Arg Ser Glu Ala Gly
    50                  55                  60

Val Thr Glu Tyr Phe Asp Glu Lys Asn Glu Leu Phe Gln Cys Thr Gly
65                  70                  75                  80

Thr Phe Val Ile Arg Arg Val Ile Gln Pro Gln Gly Leu Leu Val Pro
                85                  90                  95

Arg Tyr Thr Asn Ala Pro Gly Leu Val Tyr Ile Ile Gln Gly Arg Gly
            100                 105                 110

Ser Ile Gly Leu Thr Phe Pro Gly Cys Pro Ala Thr Tyr Gln Gln Gln
        115                 120                 125

Phe Gln Gln Phe Leu Pro Gln Glu Gln Ser Gln Ser Gln Lys Phe Arg
    130                 135                 140

Asp Glu His Gln Lys Ile His Gln Phe Arg Gln Gly Asp Ile Val Ala
145                 150                 155                 160

Leu Pro Ala Gly Val Ala His Trp Phe Tyr Asn Asp Gly Asp Ala Pro
                165                 170                 175

Val Val Ala Val Tyr Val Tyr Asp Val Lys Asn Ser Ala Asn Gln Leu
            180                 185                 190

Glu Pro Arg Gln Arg Glu Phe Leu Leu Gly Gly Asn Asn Met Arg Ala
        195                 200                 205

Gln Gln Val Tyr Gly Ser Ser Ala Glu Gln His Ser Arg Gln Asn Ile
    210                 215                 220

Phe Ser Gly Phe Gly Val Glu Ile Leu Ser Glu Ala Leu Gly Ile Ser
225                 230                 235                 240

Thr Val Thr Thr Lys Arg Leu Gln Ser Gln Asn Asp Gln Arg Gly Glu
                245                 250                 255

Ile Ile His Val Lys Asn Gly Leu Gln Phe Leu Lys Pro Thr Leu Thr
        260                 265                 270

Gln Gln Gln Glu Gln Ala Gln Ala Gln Tyr Gln Glu Val Gln Tyr Ser
    275                 280                 285

Glu Gln Gln Gln Thr Ser Ser Arg Trp Asn Gly Leu Asp Glu Asn Phe
    290                 295                 300

Cys Thr Ile Lys Ala Arg Met Asn Ile Glu Asn Thr Ser Arg Ala Asp
305                 310                 315                 320

Thr Tyr Asn Pro Arg Ala Gly Arg Thr Thr Ser Leu Asn Ser Gln Lys
                325                 330                 335

Phe Pro Ile Leu Asn Leu Val Gln Met Ser Ala Thr Arg Val Asn Leu
            340                 345                 350

Tyr Gln Asn Ala Ile Leu Ser Thr Phe Trp Asn Val Asn Ala His Ser
        355                 360                 365

Leu Val Tyr Thr Ile Gln Gly Arg Ala Arg Val Gln Val Val Ser Asn
    370                 375                 380

Phe Gly Lys Thr Val Phe Asp Gly Glu Leu Arg Pro Gly Gln Leu Leu
385                 390                 395                 400

Ile Ile Pro Gln His Tyr Val Val Leu Lys Lys Ala Gln Arg Glu Gly
                405                 410                 415

Phe Arg Tyr Ile Ala Ile Lys Thr Asn Ala Asn Ala Phe Val Ser Gln
            420                 425                 430

Leu Val Gly Lys Asn Ser Val Phe Arg Ser Leu Pro Val Asp Val Ile
        435                 440                 445

Ala Asn Val Tyr Arg Ile Ser Arg Glu Gln Ala Arg Ser Leu Lys Asn
    450                 455                 460

Asn Arg Gly Glu Glu His Gly Ala Phe Ala Pro Arg Ser Gln Gln Gln
465                 470                 475                 480

Ser Tyr Pro Gly Phe Ser Asn Gln Ser Glu Ser Glu Thr Ser Glu
                485                 490                 495

<210>  45
<211>  494
<212>  PRT

<213>  Oryza brachyantha

<400>  45

Met Ala Thr Thr Thr Phe Ser Arg Phe Ser Ile Tyr Phe Cys Val Leu
1               5                   10                  15

Leu Leu Cys His Gly Ser Met Ala Gln Leu Phe Ser Pro Thr Leu Asn
            20                  25                  30

Pro Trp His Ser Ser Arg Arg Gly Gly Ser Arg Asp Cys Arg Phe Asp
        35                  40                  45

Arg Leu Gln Ala Phe Glu Pro Leu Arg Arg Val Arg Ser Glu Ala Gly
    50                  55                  60

Val Thr Glu Tyr Phe Asp Glu Arg Asn Glu Gln Phe Gln Cys Thr Gly
65                  70                  75                  80

Thr Phe Val Ile Arg Arg Val Ile Glu Pro Gln Gly Leu Leu Val Pro
                85                  90                  95

Arg Tyr Thr Asn Thr Pro Gly Val Val Tyr Ile Met Gln Gly Arg Gly
            100                 105                 110

Ser Met Gly Leu Thr Phe Pro Gly Cys Pro Ala Thr Tyr Gln Gln Gln
        115                 120                 125

Phe Gln Gln Phe Leu Pro Glu Gly Gln Ser Gln Ser Gln Lys Phe Arg
    130                 135                 140

Asp Glu His Gln Lys Ile His Gln Phe Arg Gln Gly Asp Ile Val Ala
145                 150                 155                 160

Leu Pro Ala Gly Val Ala His Trp Phe Tyr Asn Glu Gly Asp Thr Pro
                165                 170                 175

Val Val Ala Leu Tyr Val Phe Asp Ile Asn Asn Ser Ala Asn Gln Leu
            180                 185                 190

Glu Pro Arg Gln Lys Asp Phe Leu Leu Ala Gly Asn Asn Asn Arg Glu
        195                 200                 205

Gln Gln Val Tyr Gly Arg Ser Ile Glu Lys His Ser Gly Gln Asn Ile
    210                 215                 220

Phe Ser Gly Phe Asn His Glu Leu Leu Ser Glu Ala Leu Gly Ile Ser
225                 230                 235                 240

Thr Leu Ala Ala Lys Arg Leu Gln Gly Gln Asn Asp His Arg Gly Glu
                245                 250                 255

Ile Ile Arg Val Arg Asn Gly Leu Gln Leu Leu Lys Pro Thr Phe Thr
            260                 265                 270

Gln Gln Gln Glu Gln Ala Gln Ser Gln Tyr Gln Val Gln Tyr Ser Glu
        275                 280                 285

Lys Gln Gln Glu Ser Thr Arg Cys Asn Gly Leu Asp Glu Asn Phe Cys
    290                 295                 300

Thr Ile Asn Ala Arg Leu Asn Ile Glu Asn Pro Ser Arg Ala Asp Thr
305                 310                 315                 320

Tyr Asn Pro Arg Ala Gly Arg Ile Thr His Leu Asn Asn Gln Lys Phe
                325                 330                 335

Pro Ile Leu Asn Leu Val Gln Met Ser Ala Thr Arg Val Asn Leu Tyr
            340                 345                 350

Gln Asn Ala Ile Leu Ser Pro Tyr Trp Asn Val Asn Ala His Ser Leu
        355                 360                 365

Val Tyr Met Val Gln Gly His Ala Arg Val Gln Val Val Ser Asn Leu
    370                 375                 380

Gly Lys Thr Val Phe Asn Ser Val Leu Arg Pro Gly Gln Leu Leu Ile
385                 390                 395                 400

Ile Pro Gln His Tyr Val Val Leu Lys Lys Ala Glu Arg Glu Gly Cys
                405                 410                 415

Gln Tyr Ile Ala Phe Lys Thr Asn Ala Asn Ser Ile Val Ser Gln Leu
            420                 425                 430

Ala Gly Lys Asn Ser Ile Leu Arg Ala Met Pro Val Asp Val Val Ala
        435                 440                 445

Asn Ala Tyr Arg Ile Ser Arg Glu Gln Ala Arg Asp Leu Lys Asn Asn
    450                 455                 460

Arg Gly Glu Glu Leu Gly Ala Phe Thr Pro Lys Phe Glu Gln Gln Ser
465                 470                 475                 480

Tyr Pro Gly Leu Ser Asn Glu Ser Glu Ser Glu Ala Ser Glu
                485                 490

<210>  46
<211>  500
<212>  PRT
<213>  Zizania latifolia

<400>  46

Met Asn Met Ala Thr Ile Asn Gly Pro Thr Ile Phe Phe Thr Val Cys
1               5                   10                  15

Leu Phe Leu Leu Cys His Gly Ser Leu Ala Gln Leu Leu Gly Gln Ser
            20                  25                  30

Thr Ser Gln Trp Gln Ser Ser His Arg Gly Ser Ser Arg Gln Cys Arg
        35                  40                  45

Phe Asp Arg Leu Gln Ala Phe Glu Pro Val Arg Ser Val Arg Ser Gln
    50                  55                  60

Ala Gly Thr Thr Glu Phe Phe Asp Ala Ser Asn Glu Leu Phe Gln Cys
65                  70                  75                  80

Ala Gly Val Ser Ile Val Arg Arg Ile Ile Glu Pro Arg Gly Leu Leu
                85                  90                  95

Leu Pro Gln Tyr Thr Asn Gly Ala Thr Ile Met Tyr Ile Ile Gln Gly
            100                 105                 110

Arg Gly Ile Thr Gly Gln Thr Phe Pro Gly Cys Pro Glu Ser Tyr Gln
        115                 120                 125

Gln Gln Phe Gln Gln Ser Met Gln Ala Gln Leu Thr Gly Ser Gln Ser
    130                 135                 140

Gln Ser Gln Lys Phe Lys Asp Glu His Gln Lys Ile Asn Arg Phe Arg
145                 150                 155                 160

Gln Gly Asp Val Ile Ala Leu Pro Ala Gly Val Ala His Trp Cys Tyr
                165                 170                 175

Asn Asp Gly Glu Val Pro Val Val Ala Ile Tyr Val Ile Asp Ile Asn
            180                 185                 190

Asn Ala Ala Asn Gln Leu Asp Pro Arg Gln Arg Asp Phe Leu Leu Ala
        195                 200                 205

Gly Asn Met Arg Ser Pro Gln Ala Tyr Arg Arg Glu Val Glu Asn Gln
    210                 215                 220

Ser Gln Asn Ile Phe Ser Gly Phe Ser Ala Glu Leu Leu Ser Glu Ala
225                 230                 235                 240

Leu Gly Ile Ser Thr Gly Val Ala Arg Gln Leu Gln Cys Gln Asn Asp
                245                 250                 255

Gln Arg Gly Glu Ile Val Arg Val Glu His Gly Leu Ser Leu Leu Gln
            260                 265                 270

Pro Tyr Ala Ser Leu Gln Glu Gln Glu Gln Lys Gln Glu Gln Pro Arg
        275                 280                 285

Glu Arg Tyr Gln Val Thr Gln His Gln Gln Ser Gln Tyr Gly Gly Gly
    290                 295                 300

Cys Ser Asn Gly Leu Asp Glu Thr Phe Cys Ala Met Arg Ile Trp Gln
305                 310                 315                 320

Asn Ile Asp Asn Pro Asn Leu Ala Asp Thr Tyr Asn Pro Arg Ala Gly
                325                 330                 335

Arg Val Thr Asn Leu Asn Ser Gln Lys Phe Pro Ile Leu Asn Leu Ile
            340                 345                 350

Gln Met Ser Ala Val Lys Val Asn Leu Tyr Gln Asn Ala Leu Leu Ser
        355                 360                 365

Pro Phe Trp Asn Ile Asn Ser His Ser Val Val Tyr Val Thr Gln Gly
    370                 375                 380

Cys Ala Arg Val Gln Val Val Asn Asn Asn Gly Lys Thr Val Phe Asn
385                 390                 395                 400

Gly Glu Leu Arg Arg Gly Gln Leu Leu Ile Ile Pro Gln His Tyr Val
                405                 410                 415

Val Val Lys Lys Ala Gln Arg Glu Gly Cys Ala Tyr Ile Ala Phe Lys
            420                 425                 430

Thr Asn Pro Asn Ser Met Val Ser His Ile Val Gly Lys Ser Ser Ile
        435                 440                 445

Phe Arg Ala Leu Pro Thr Asp Val Leu Ala Asn Ala Tyr Arg Ile Ser
    450                 455                 460

Arg Glu Asp Ala Gln Arg Leu Lys His Asn Arg Gly Asp Glu Leu Gly

465                 470                 475                 480

Ala Phe Thr Pro Leu Gln Tyr Lys Ser Tyr Gln Asp Val Ser Ser Val
                485                 490                 495

Ala Ala Ser Ser
            500

<210>  47
<211>  495
<212>  PRT
<213>  Oryza sativa

<400>  47

Met Thr Ile Ser Val Phe Ser Arg Phe Ser Ile Tyr Phe Cys Val Leu
1               5                   10                  15

Leu Leu Cys Asn Gly Ser Met Ala Gln Leu Phe Asp Pro Ala Thr Asn
            20                  25                  30

Gln Trp Gln Thr His Arg Gln Gly Ser Phe Arg Glu Cys Arg Phe Glu
        35                  40                  45

Arg Leu Gln Ala Phe Glu Pro Leu Gln Asn Val Arg Ser Glu Ala Gly
    50                  55                  60

Val Thr Glu Tyr Phe Asp Glu Thr Asn Glu Leu Phe Gln Cys Thr Gly
65                  70                  75                  80

Thr Phe Val Ile Arg Arg Val Ile Gln Pro Gln Gly Leu Leu Ile Pro
                85                  90                  95

Arg Tyr Ala Asn Thr Pro Gly Met Val Tyr Ile Ile Gln Gly Arg Gly
            100                 105                 110

Ser Met Gly Leu Thr Phe Pro Gly Cys Pro Ala Thr Tyr Gln Gln Gln
        115                 120                 125

Ser Gln Gln Phe Leu Phe Gln Gly Glu Ser Gln Ser Gln Lys Phe Ile
    130                 135                 140

Asp Glu His Gln Lys Ile His Gln Phe Arg Gln Gly Asp Ile Val Val
145                 150                 155                 160

Leu Pro Thr Gly Val Ala His Trp Phe Tyr Asn Asp Gly Asp Thr Pro
                165                 170                 175

Val Val Ala Leu Tyr Val Tyr Asp Ile Asn Asn Ser Ala Asn Gln Leu

180                 185                 190

Glu Pro Arg His Arg Glu Phe Leu Leu Ala Gly Lys Asn Asn Arg Val
        195                 200                 205

Gln Gln Val Tyr Gly Arg Ser Ile Gln Gln His Ser Gly Gln Asn Ile
    210                 215                 220

Phe Asn Gly Phe Ser Val Glu Pro Leu Ser Glu Ala Leu Asn Ile Asn
225                 230                 235                 240

Thr Val Thr Thr Lys Arg Leu Gln Ser Gln Asn Asp Gln Arg Gly Glu
                245                 250                 255

Ile Ile His Val Lys Asn Gly Leu Gln Leu Leu Lys Pro Thr Leu Thr
            260                 265                 270

Gln Arg Gln Glu Gln Glu Gln Ala Gln Tyr Gln Glu Val Gln Tyr Ser
        275                 280                 285

Glu Lys Pro Gln Thr Ser Ser Arg Trp Asn Gly Leu Glu Glu Asn Leu
    290                 295                 300

Cys Thr Ile Lys Thr Arg Leu Asn Ile Glu Asn Pro Ser Arg Ala Asp
305                 310                 315                 320

Ser Tyr Asp Pro Arg Ala Gly Arg Ile Thr Ser Leu Asp Ser Gln Lys
                325                 330                 335

Phe Pro Ile Leu Asn Ile Ile Gln Met Ser Ala Thr Arg Val Asn Leu
            340                 345                 350

Tyr Gln Asn Ala Ile Leu Thr Pro Phe Trp Asn Val Asn Ala His Ser
        355                 360                 365

Leu Met Tyr Val Ile Arg Gly Arg Ala Arg Val Gln Val Val Ser Asn
    370                 375                 380

Phe Gly Lys Thr Val Phe Asp Gly Val Leu Arg Pro Glu Gln Leu Leu
385                 390                 395                 400

Ile Ile Pro Gln Asn Tyr Val Val Leu Lys Lys Ala Gln His Glu Gly
                405                 410                 415

Cys Gln Tyr Ile Ala Ile Asn Thr Asn Ala Asn Ala Phe Val Ser His
            420                 425                 430

Leu Ala Gly Val Asp Ser Val Phe His Ala Leu Pro Val Asp Val Ile
        435                 440                 445

Ala Asn Ala Tyr Cys Ile Ser Arg Glu Glu Ala Arg Arg Leu Lys Asn
    450                 455                 460

Asn Arg Gly Asp Glu Tyr Gly Pro Phe Pro Pro Arg Leu Gln Gln Gln
465                 470                 475                 480

Ile Tyr Pro Glu Phe Ser Asn Glu Ser Lys Gly Glu Thr Ser Glu
                485                 490                 495

<210>  48
<211>  480
<212>  PRT
<213>  Oryza sativa

<400>  48

Leu Leu Cys His Gly Ser Met Ala Gln Ile Phe Ser Leu Gly Ile Asn
1               5                   10                  15

Pro Trp Gln Asn Pro Arg Gln Gly Gly Ser Arg Glu Cys Arg Phe Asp
            20                  25                  30

Arg Leu Gln Ala Phe Glu Pro Leu Arg Lys Val Arg His Glu Ala Gly
        35                  40                  45

Val Thr Glu Tyr Phe Asp Glu Lys Asn Glu Gln Phe Gln Cys Thr Gly
    50                  55                  60

Thr Leu Val Ile Arg Arg Ile Ile Glu Pro Gln Gly Leu Leu Leu Pro
65                  70                  75                  80

Arg Tyr Ser Asn Thr Pro Gly Leu Val Tyr Ile Ile Gln Gly Thr Gly
                85                  90                  95

Val Leu Gly Leu Thr Phe Pro Gly Cys Pro Ala Thr Tyr Gln Lys Gln
            100                 105                 110

Phe Arg His Phe Gly Leu Glu Gly Gly Ser Gln Arg Gln Gly Lys Lys
        115                 120                 125

Leu Arg Asp Glu Asn Gln Lys Ile His Gln Phe Arg Gln Gly Asp Val
    130                 135                 140

Val Ala Leu Pro Ser Gly Ile Pro His Trp Phe Tyr Asn Glu Gly Asp
145                 150                 155                 160

Thr Pro Val Val Ala Leu Phe Val Phe Asp Val Asn Asn Asn Ala Asn
                165                 170                 175

Gln Leu Glu Pro Arg Gln Lys Glu Phe Leu Leu Ala Gly Asn Asn Ile
            180                 185                 190

Glu Gln Gln Val Ser Asn Pro Ser Ile Asn Lys His Ser Gly Gln Asn
        195                 200                 205

Ile Phe Asn Gly Phe Asn Thr Lys Leu Leu Ser Glu Ala Leu Gly Val
    210                 215                 220

Asn Ile Glu Val Thr Arg Arg Leu Gln Ser Gln Asn Asp Arg Arg Gly
225                 230                 235                 240

Asp Ile Ile Arg Val Lys Asn Gly Leu Arg Leu Ile Lys Pro Thr Ile
                245                 250                 255

Thr Gln Gln Gln Glu Gln Thr Gln Asp Gln Tyr Gln Gln Ile Gln Tyr
            260                 265                 270

His Arg Glu Gln Arg Ser Thr Ser Lys Tyr Asn Gly Leu Asp Glu Asn
        275                 280                 285

Phe Cys Ala Ile Arg Ala Arg Leu Asn Ile Glu Asn Pro Asn His Ala
    290                 295                 300

Asp Thr Tyr Asn Pro Arg Ala Gly Arg Ile Thr Asn Leu Asn Ser Gln
305                 310                 315                 320

Lys Phe Ser Ile Leu Asn Leu Val Gln Met Ser Ala Thr Arg Val Asn
                325                 330                 335

Leu Tyr Gln Asn Ala Ile Leu Ser Pro Phe Trp Asn Ile Asn Ala His
            340                 345                 350

Ser Leu Val Tyr Thr Ile Gln Gly Arg Ala Arg Val Gln Val Val Ser
        355                 360                 365

Asn His Gly Lys Ala Val Phe Asn Gly Val Leu Arg Pro Gly Gln Leu
    370                 375                 380

Leu Ile Ile Pro Gln Asn Tyr Val Val Met Lys Lys Ala Glu Leu Glu
385                 390                 395                 400

Gly Phe Gln Phe Ile Ala Phe Lys Thr Asn Pro Asn Ala Met Val Asn
                405                 410                 415

His Ile Ala Gly Lys Asn Ser Val Leu Arg Ala Met Pro Val Asp Val
            420                 425                 430

Ile Ala Asn Ala Tyr Arg Ile Ser Arg Gln Glu Ala Arg Ser Leu Lys
        435                 440                 445

Asn Asn Arg Gly Glu Glu Ile Gly Ala Phe Thr Pro Arg Tyr Gln Gln
    450                 455                 460

Gln Lys Ile His Gln Glu Tyr Ser Asn Pro Asn Glu Ser Glu Thr Gln
465                 470                 475                 480

<210>  49
<211>  518
<212>  PRT
<213>  Avena sativa

<400>  49

Met Ala Thr Thr Arg Phe Pro Ser Leu Leu Phe Tyr Ser Cys Ile Phe
1               5                   10                  15

Leu Leu Cys Asn Gly Ser Met Ala Gln Leu Phe Gly Gln Ser Phe Thr
            20                  25                  30

Pro Trp Gln Ser Ser Arg Gln Gly Gly Leu Arg Gly Cys Arg Phe Asp
        35                  40                  45

Arg Leu Gln Ala Phe Glu Pro Leu Arg Gln Val Arg Ser Gln Ala Gly
    50                  55                  60

Ile Thr Glu Tyr Phe Asp Glu Gln Asn Glu Gln Phe Arg Cys Ala Gly
65                  70                  75                  80

Val Ser Val Ile Arg Arg Val Ile Glu Pro Gln Gly Leu Leu Leu Pro
                85                  90                  95

Gln Tyr His Asn Ala Pro Gly Leu Val Tyr Ile Leu Gln Gly Arg Gly
            100                 105                 110

Phe Thr Gly Leu Thr Phe Pro Gly Cys Pro Ala Thr Phe Gln Gln Gln
        115                 120                 125

Phe Gln Pro Phe Asp Gln Ala Arg Phe Ala Gln Gly Gln Ser Lys Ser
    130                 135                 140

Gln Asn Leu Lys Asp Glu His Gln Arg Val His His Ile Lys Gln Gly
145                 150                 155                 160

Asp Val Val Ala Leu Pro Ala Gly Ile Val His Trp Cys Tyr Asn Asp
                165                 170                 175

Gly Asp Ala Pro Ile Val Ala Val Tyr Val Phe Asp Val Asn Asn Asn
            180                 185                 190

Ala Asn Gln Leu Glu Pro Arg Gln Lys Glu Phe Leu Leu Ala Gly Asn
        195                 200                 205

Asn Lys Arg Glu Gln Gln Phe Gly Gln Asn Ile Phe Ser Gly Phe Ser
    210                 215                 220

Val Gln Leu Leu Ser Glu Ala Leu Gly Ile Ser Gln Gln Ala Ala Gln
225                 230                 235                 240

Lys Ile Gln Ser Gln Asn Asp Gln Arg Gly Glu Ile Ile Arg Val Ser
                245                 250                 255

Gln Gly Leu Gln Phe Leu Lys Pro Phe Val Ser Gln Gln Gly Pro Val
            260                 265                 270

Glu His Gln Ala Tyr Gln Pro Ile Gln Ser Gln Gln Glu Gln Ser Thr
        275                 280                 285

Gln Tyr Gln Val Gly Gln Ser Pro Gln Tyr Gln Glu Gly Gln Ser Thr
    290                 295                 300

Gln Tyr Gln Ser Gly Gln Ser Trp Asp Gln Ser Phe Asn Gly Leu Glu
305                 310                 315                 320

Glu Asn Phe Cys Ser Leu Glu Ala Arg Gln Asn Ile Glu Asn Pro Lys
                325                 330                 335

Arg Ala Asp Thr Tyr Asn Pro Arg Ala Gly Arg Ile Thr His Leu Asn
            340                 345                 350

Ser Lys Asn Phe Pro Thr Leu Asn Leu Val Gln Met Ser Ala Thr Arg
        355                 360                 365

Val Asn Leu Tyr Gln Asn Ala Ile Leu Ser Pro Tyr Trp Asn Ile Asn
    370                 375                 380

Ala His Ser Val Met His Met Ile Gln Gly Arg Ala Arg Val Gln Val
385                 390                 395                 400

Val Asn Asn His Gly Gln Thr Val Phe Asn Asp Ile Leu Arg Arg Gly
                405                 410                 415

Gln Leu Leu Ile Ile Pro Gln His Tyr Val Val Leu Lys Lys Ala Glu
            420                 425                 430

Arg Glu Gly Cys Gln Tyr Ile Ser Phe Lys Thr Thr Pro Asn Ser Met
        435                 440                 445

Val Ser Tyr Ile Ala Gly Lys Thr Ser Ile Leu Arg Ala Leu Pro Val
    450                 455                 460

Asp Val Leu Ala Asn Ala Tyr Arg Ile Ser Arg Gln Glu Ser Gln Asn
465                 470                 475                 480

Leu Lys Asn Asn Arg Gly Glu Glu Phe Gly Ala Phe Thr Pro Lys Phe
                485                 490                 495

Ala Gln Thr Gly Ser Gln Ser Tyr Gln Asp Glu Gly Glu Ser Ser Ser
            500                 505                 510

Thr Glu Lys Ala Ser Glu
        515

<210>  50
<211>  454
<212>  PRT
<213>  Oryza sativa

<400>  50

Cys Arg Phe Asp Arg Leu Gln Ala Phe Glu Pro Ile Arg Ser Val Arg
1               5                   10                  15

Ser Gln Ala Gly Thr Thr Glu Phe Phe Asp Val Ser Asn Glu Gln Phe
            20                  25                  30

Gln Cys Thr Gly Val Ser Ala Val Arg Arg Val Ile Glu Pro Arg Gly
        35                  40                  45

Leu Leu Leu Pro His Tyr Thr Asn Gly Ala Ser Leu Val Tyr Ile Ile
    50                  55                  60

Gln Gly Arg Gly Ile Thr Gly Pro Thr Phe Pro Gly Cys Pro Glu Ser
65                  70                  75                  80

Tyr Gln Gln Gln Phe Gln Gln Ser Gly Gln Ala Gln Leu Thr Glu Ser
                85                  90                  95

Gln Ser Gln Ser His Lys Phe Lys Asp Glu His Gln Lys Ile His Arg
            100                 105                 110

Phe Arg Gln Gly Asp Val Ile Ala Leu Pro Ala Gly Val Ala His Trp
        115                 120                 125

Cys Tyr Asn Asp Gly Glu Val Pro Val Val Ala Ile Tyr Val Thr Asp
    130                 135                 140

Leu Asn Asn Gly Ala Asn Gln Leu Asp Pro Arg Gln Arg Asp Phe Leu
145                 150                 155                 160

Leu Ala Gly Asn Lys Arg Asn Pro Gln Ala Tyr Arg Arg Glu Val Glu
                165                 170                 175

Glu Arg Ser Gln Asn Ile Phe Ser Gly Phe Ser Thr Glu Leu Leu Ser
            180                 185                 190

Glu Ala Leu Gly Val Ser Ser Gln Val Ala Arg Gln Leu Gln Cys Gln
        195                 200                 205

Asn Asp Gln Arg Gly Glu Ile Val Arg Val Glu His Gly Leu Ser Leu
    210                 215                 220

Leu Gln Pro Tyr Ala Ser Leu Gln Glu Gln Glu Gln Gly Gln Val Gln
225                 230                 235                 240

Ser Arg Glu Arg Tyr Gln Glu Gly Gln Tyr Gln Gln Ser Gln Tyr Gly
                245                 250                 255

Ser Gly Cys Ser Asn Gly Leu Asp Glu Thr Phe Cys Thr Met Lys Val
            260                 265                 270

Arg Gln Asn Ile Asp Asn Pro Asn Arg Ala Asp Thr Tyr Asn Pro Arg
        275                 280                 285

Ala Gly Arg Val Thr Asn Leu Asn Thr Gln Asn Phe Pro Ile Leu Asn
    290                 295                 300

Leu Val Gln Met Ser Ala Val Lys Val Asn Leu Tyr Gln Asn Ala Leu
305                 310                 315                 320

Leu Ser Pro Phe Trp Asn Ile Asn Ala His Ser Val Val Tyr Ile Thr
                325                 330                 335

Gln Gly Arg Ala Arg Val Gln Val Val Asn Asn Asn Gly Lys Thr Val
            340                 345                 350

Phe Asn Gly Glu Leu Arg Arg Gly Gln Leu Leu Ile Ile Pro Gln His

355                 360                 365

Tyr Ala Val Val Lys Lys Ala Gln Arg Glu Gly Cys Ala Tyr Ile Ala
    370                 375                 380

Phe Lys Thr Asn Pro Asn Ser Met Val Ser His Ile Ala Gly Lys Ser
385                 390                 395                 400

Ser Ile Phe Arg Ala Leu Pro Asn Asp Val Leu Ala Asn Ala Tyr Arg
                405                 410                 415

Ile Ser Arg Glu Glu Ala Gln Arg Leu Lys His Asn Arg Gly Asp Glu
            420                 425                 430

Phe Gly Ala Phe Thr Pro Ile Gln Tyr Lys Ser Tyr Gln Asp Val Tyr
        435                 440                 445

Asn Ala Ala Glu Ser Ser
    450

<210>  51
<211>  500
<212>  PRT
<213>  Zizania latifolia

<400>  51

Met Asn Met Ala Thr Ile Asn Gly Pro Thr Ile Phe Phe Thr Val Cys
1               5                   10                  15

Leu Phe Leu Leu Cys His Gly Ser Leu Ala Gln Leu Leu Gly Gln Ser
            20                  25                  30

Thr Ser Gln Trp Gln Ser Ser His Arg Gly Ser Ser Arg Gln Cys Arg
        35                  40                  45

Phe Asp Arg Leu Gln Ala Phe Glu Pro Val Arg Ser Val Arg Ser Gln
    50                  55                  60

Ala Gly Thr Thr Glu Phe Phe Asp Ala Ser Asn Glu Leu Phe Gln Cys
65                  70                  75                  80

Ala Gly Val Ser Ile Val Arg Arg Ile Ile Glu Pro Arg Gly Leu Leu
                85                  90                  95

Leu Pro Gln Tyr Thr Asn Gly Ala Thr Ile Met Tyr Ile Ile Gln Gly
            100                 105                 110

Arg Gly Ile Thr Gly Gln Thr Phe Pro Gly Cys Pro Glu Ser Tyr Gln

115                 120                 125

Gln Gln Phe Gln Gln Ser Met Gln Ala Gln Leu Thr Gly Ser Gln Ser
    130                 135                 140

Gln Ser Gln Lys Phe Lys Asp Glu His Gln Lys Ile Asn Arg Phe Arg
145                 150                 155                 160

Gln Gly Asp Val Ile Ala Leu Pro Ala Gly Val Ala His Trp Cys Tyr
                165                 170                 175

Asn Asp Gly Glu Val Pro Val Val Ala Ile Tyr Val Ile Asp Ile Asn
            180                 185                 190

Asn Ala Ala Asn Gln Leu Asp Pro Arg Gln Arg Asp Phe Leu Leu Ala
        195                 200                 205

Gly Asn Met Arg Ser Pro Gln Ala Tyr Arg Arg Glu Val Glu Asn Gln
    210                 215                 220

Ser Gln Asn Ile Phe Ser Gly Phe Ser Ala Glu Leu Leu Ser Glu Ala
225                 230                 235                 240

Leu Gly Ile Ser Thr Gly Val Ala Arg Gln Leu Gln Cys Gln Asn Asp
                245                 250                 255

Gln Arg Gly Glu Ile Val Arg Val Glu His Gly Leu Ser Leu Leu Gln
            260                 265                 270

Pro Tyr Ala Ser Leu Gln Glu Gln Glu Gln Lys Gln Glu Gln Pro Arg
        275                 280                 285

Glu Arg Tyr Gln Val Thr Gln His Gln Gln Ser Gln Tyr Gly Gly Gly
    290                 295                 300

Cys Ser Asn Gly Leu Asp Glu Thr Phe Cys Ala Met Arg Ile Trp Gln
305                 310                 315                 320

Asn Ile Asp Asn Pro Asn Leu Ala Asp Thr Tyr Asn Pro Arg Ala Gly
                325                 330                 335

Arg Val Thr Asn Leu Asn Ser Gln Lys Phe Pro Ile Leu Asn Leu Ile
            340                 345                 350

Gln Met Ser Ala Val Lys Val Asn Leu Tyr Gln Asn Ala Leu Leu Ser
        355                 360                 365

Pro Phe Trp Asn Ile Asn Ser His Ser Val Val Tyr Val Thr Gln Gly
    370                 375                 380

Cys Ala Arg Val Gln Val Val Asn Asn Asn Gly Lys Thr Val Phe Asn
385                 390                 395                 400

Gly Glu Leu Arg Arg Gly Gln Leu Leu Ile Ile Pro Gln His Tyr Val
                405                 410                 415

Val Val Lys Lys Ala Gln Arg Glu Gly Cys Ala Tyr Ile Ala Phe Lys
            420                 425                 430

Thr Asn Pro Asn Ser Met Val Ser His Ile Val Gly Lys Ser Ser Ile
        435                 440                 445

Phe Arg Ala Leu Pro Thr Asp Val Leu Ala Asn Ala Tyr Arg Ile Ser
    450                 455                 460

Arg Glu Asp Ala Gln Arg Leu Lys His Asn Arg Gly Asp Glu Leu Gly
465                 470                 475                 480

Ala Phe Thr Pro Leu Gln Tyr Lys Ser Tyr Gln Asp Val Ser Ser Val
                485                 490                 495

Ala Ala Ser Ser
            500

<210>  52
<211>  527
<212>  PRT
<213>  Avena sativa

<400>  52

Met Ala Thr Thr Ser Phe Pro Ser Met Leu Phe Tyr Phe Cys Ile Phe
1               5                   10                  15

Leu Leu Phe His Gly Ser Met Ala Gln Leu Phe Gly Gln Ser Ser Thr
            20                  25                  30

Pro Trp Gln Ser Ser Arg Gln Gly Gly Leu Arg Gly Cys Arg Phe Asp
        35                  40                  45

Arg Leu Gln Ala Phe Glu Pro Leu Arg Gln Val Arg Ser Gln Ala Gly
    50                  55                  60

Ile Thr Glu Tyr Phe Asp Glu Gln Asn Glu Gln Phe Arg Cys Thr Gly
65                  70                  75                  80

Val Ser Val Ile Arg Arg Val Ile Glu Pro Gln Gly Leu Val Leu Pro
                85                  90                  95

Gln Tyr His Asn Ala Pro Ala Leu Val Tyr Ile Leu Gln Gly Arg Gly
            100                 105                 110

Phe Thr Gly Leu Thr Phe Pro Gly Cys Pro Ala Thr Phe Gln Gln Gln
        115                 120                 125

Phe Gln Pro Phe Asp Gln Ser Gln Phe Ala Gln Gly Gln Arg Gln Ser
    130                 135                 140

Gln Thr Ile Lys Asp Glu His Gln Arg Val Gln Arg Phe Lys Gln Gly
145                 150                 155                 160

Asp Val Val Ala Leu Pro Ala Gly Ile Val His Trp Cys Tyr Asn Asp
                165                 170                 175

Gly Asp Ala Pro Ile Val Ala Ile Tyr Val Phe Asp Val Asn Asn Asn
            180                 185                 190

Ala Asn Gln Leu Glu Pro Arg Gln Lys Glu Phe Leu Leu Ala Gly Asn
        195                 200                 205

Asn Lys Arg Glu Gln Gln Ser Gly Asn Asn Ile Phe Ser Gly Leu Ser
    210                 215                 220

Val Gln Leu Leu Ser Glu Ala Leu Gly Ile Ser Gln Gln Ala Ala Gln
225                 230                 235                 240

Arg Ile Gln Ser Gln Asn Asp Gln Arg Gly Glu Ile Ile Arg Val Ser
                245                 250                 255

Gln Gly Leu Gln Phe Leu Lys Pro Ile Val Ser Gln Gln Val Pro Gly
            260                 265                 270

Glu Gln Gln Val Tyr Gln Pro Ile Gln Thr Gln Glu Gly Gln Ala Thr
        275                 280                 285

Gln Tyr Gln Val Gly Gln Ser Thr Gln Tyr Gln Val Gly Lys Ser Thr
    290                 295                 300

Pro Tyr Gln Gly Gly Gln Ser Ser Gln Tyr Gln Ala Gly Gln Ser Trp
305                 310                 315                 320

Asp Gln Ser Phe Asn Gly Leu Glu Glu Asn Phe Cys Ser Leu Glu Ala
                325                 330                 335

Arg Lys Asn Ile Glu Asn Pro Gln His Ala Asp Thr Tyr Asn Pro Arg
            340                 345                 350

Ala Gly Arg Ile Thr Arg Leu Asn Ser Lys Asn Phe Pro Ile Leu Asn
        355                 360                 365

Ile Val Gln Met Ser Ala Thr Arg Val Asn Leu Tyr Gln Asn Ala Ile
    370                 375                 380

Leu Ser Pro Phe Trp Asn Ile Asn Ala His Ser Val Ile Tyr Met Ile
385                 390                 395                 400

Gln Gly His Ala Arg Val Gln Val Val Asn Asn Asn Gly Gln Thr Val
                405                 410                 415

Phe Asn Asp Ile Leu Arg Arg Gly Gln Leu Leu Ile Val Pro Gln His
            420                 425                 430

Phe Val Val Leu Lys Lys Ala Glu Arg Glu Gly Cys Gln Tyr Ile Ser
        435                 440                 445

Phe Lys Thr Asn Pro Asn Ser Met Val Ser His Ile Ala Gly Lys Ser
    450                 455                 460

Ser Ile Leu Arg Ala Leu Pro Ile Asp Val Leu Ala Asn Ala Tyr Arg
465                 470                 475                 480

Ile Ser Arg Gln Glu Ala Arg Asn Leu Lys Asn Asn Arg Gly Glu Glu
                485                 490                 495

Phe Gly Ala Phe Thr Pro Lys Leu Thr Gln Lys Gly Phe Gln Ser Tyr
            500                 505                 510

Gln Asp Ile Glu Glu Gly Ser Ser Ser Pro Val Arg Ala Ser Glu
        515                 520                 525

<210> 53
<211> 463
<212> PRT
<213> Vicia faba

<400> 53

Met Ala Ala Thr Thr Leu Lys Asp Ser Phe Pro Leu Leu Thr Leu Leu
1               5                   10                  15

Gly Ile Ala Phe Leu Ala Ser Val Cys Leu Ser Ser Arg Ser Asp Gln
            20                  25                  30

Asp Asn Pro Phe Val Phe Glu Ser Asn Arg Phe Gln Thr Leu Phe Glu
        35                  40                  45

Asn Glu Asn Gly His Ile Arg Leu Leu Gln Lys Phe Asp Gln His Ser
    50                  55                  60

Lys Leu Leu Glu Asn Leu Gln Asn Tyr Arg Leu Leu Glu Tyr Lys Ser
65                  70                  75                  80

Lys Pro His Thr Ile Phe Leu Pro Gln Gln Thr Asp Ala Asp Phe Ile
                85                  90                  95

Leu Val Val Leu Ser Gly Lys Ala Ile Leu Thr Val Leu Leu Pro Asn
            100                 105                 110

Asp Arg Asn Ser Phe Ser Leu Glu Arg Gly Asp Thr Ile Lys Leu Pro
        115                 120                 125

Ala Gly Thr Ile Gly Tyr Leu Val Asn Arg Asp Asp Glu Glu Asp Leu
    130                 135                 140

Arg Val Leu Asp Leu Val Ile Pro Val Asn Arg Pro Gly Glu Pro Gln
145                 150                 155                 160

Ser Phe Leu Leu Ser Gly Asn Gln Asn Gln Pro Ser Ile Leu Ser Gly
                165                 170                 175

Phe Ser Lys Asn Ile Leu Glu Ala Ser Phe Asn Thr Asp Tyr Lys Glu
            180                 185                 190

Ile Glu Lys Val Leu Leu Glu Glu His Gly Lys Glu Lys Tyr His Arg
        195                 200                 205

Arg Gly Leu Lys Asp Arg Arg Gln Arg Gly Gln Glu Glu Asn Val Ile
    210                 215                 220

Val Lys Ile Ser Arg Lys Gln Ile Glu Glu Leu Asn Lys Asn Ala Lys
225                 230                 235                 240

Ser Ser Ser Lys Lys Ser Thr Ser Ser Glu Ser Glu Pro Phe Asn Leu
                245                 250                 255

Arg Ser Arg Glu Pro Ile Tyr Ser Asn Lys Phe Gly Lys Phe Phe Glu
            260                 265                 270

Ile Thr Pro Lys Arg Asn Pro Gln Leu Gln Asp Leu Asn Ile Phe Val
        275                 280                 285

Asn Tyr Val Glu Ile Asn Glu Gly Ser Leu Leu Leu Pro His Tyr Asn
    290                 295                 300

Ser Arg Ala Ile Val Ile Val Thr Val Asn Glu Gly Lys Gly Asp Phe
305                 310                 315                 320

Glu Leu Val Gly Gln Arg Asn Glu Asn Gln Gln Gly Leu Arg Glu Glu
                325                 330                 335

Tyr Asp Glu Glu Lys Glu Gln Gly Glu Glu Glu Ile Arg Lys Gln Val
            340                 345                 350

Gln Asn Tyr Lys Ala Lys Leu Ser Pro Gly Asp Val Leu Val Ile Pro
        355                 360                 365

Ala Gly Tyr Pro Val Ala Ile Lys Ala Ser Ser Asn Leu Asn Leu Val
    370                 375                 380

Gly Phe Gly Ile Asn Ala Glu Asn Asn Gln Arg Tyr Phe Leu Ala Gly
385                 390                 395                 400

Glu Glu Asp Asn Val Ile Ser Gln Ile His Lys Pro Val Lys Glu Leu
                405                 410                 415

Ala Phe Pro Gly Ser Ala Gln Glu Val Asp Thr Leu Leu Glu Asn Gln
            420                 425                 430

Lys Gln Ser His Phe Ala Asn Ala Gln Pro Arg Glu Arg Glu Arg Gly
        435                 440                 445

Ser Gln Glu Ile Lys Asp His Leu Tyr Ser Ile Leu Gly Ser Phe
    450                 455                 460

## Description

### Background to the Invention

Hundreds of millions of people are affected each year by serious bacterial infections (WHO, 2014). Many die from these infections due to antibiotic resistance (WHO, 2014). Antibiotics are not only used heavily in the treatment of human bacterial infections but are also used in the animal farming area to maintain the health of many animals. As antibiotic resistance increases globally, the use of antibiotics for both human and animal health is of major concern and will have major implications in the 21^{st} century. Indeed, the WHO indicates in their 2014 report on antimicrobial resistance that "resistance to common bacteria has reached alarming levels in many parts of the world and in some settings, few, if any, of the available treatments options remain effective for common infections" (WHO, 2014).

Most of the current antibiotics on the market function within bacteria after penetrating the bacterial cell. The antibiotic performs its function through the inhibition of translation, the inhibition of DNA synthesis, or the inhibition of cell wall synthesis. Two examples of antibiotics used today include tetracycline, which inhibits translation within the bacterial cell by binding to ribosomes, and Penicillin, which inhibits cell wall synthesis.

However, most antibiotic mechanisms of action create bacterial resistance. The bacterium mutates and becomes resistant to an antibiotic by either changing its wall permeability, changing the receptor's 3-D structure, or, literally, destroying the antibiotic.

Besides the above, most of the current antibiotics have drastic side effects such as allergic reactions, nausea, diarrhoea, stomach pain, fever, headache and even kidney and liver damage. Therefore, there is a clear and growing need for the identification of new agents that have an anti-microbial activity with low side effects and that can ultimately cause little or no bacterial resistance.

Anti-microbial peptides (AMP) have a much broader action compared to other antibiotics. These peptides usually bind by electrostatic forces to, for example, charged lipids or amino acids on the membrane, causing perforation of the membrane. The inner part of the bacterial cell is thus exposed to external pressure and conditions and undergoes lyses. The perforation action of an anti-microbial peptide makes it very hard for the bacteria to build resistance as it is being destroyed from the outside rather than the inside.

On one hand our body already uses bioactive peptides to fight Gram-positive and Gram-negative bacteria, fungi, parasites and some viruses as a part of the body's innate immunity (Gordon Y.J. *et al.,* 2005). On the other hand, it has been shown that some foods such as milk contain some very interesting peptides with AM activity (Dallas D.C., *et al.,* 2013). We set out to identify food derived anti-microbial peptides, which, because our bodies are already accustomed to processing food molecules, are safer and as effective as antibiotics. The peptides identified can be used in many different products such as personal care, supplements, pharmaceuticals and food preservatives. In personal care for instance, these peptides can be used as an anti-odour, in pharmaceuticals they can be used to treat infections, and in food, they can be used as preservatives. These peptides also have uses as biocides in hospital settings and in agriculture to protect plants.

It is an object of the invention to provide a natural, food grade, antibacterial agent.

### Statements of Invention

The pea genome codes for over 70,000 different proteins. The Applicant has identified three of these proteins, each of which contains one or more peptides capable of inhibiting growth of bacteria (hereafter "antibacterial peptide" or "antibacterial fragment"). Likewise, out of the more than 60,000 proteins encoded by the rice genome, the Applicant has identified three proteins, each of which contains one or more peptides capable of inhibiting growth of bacteria. Antibacterial fragments of the six identified proteins, and compositions containing a plurality of the antibacterial peptides have been shown to inhibit bacterial growth in agar plates. The specific plant proteins from which the natural peptides are derived are provided in SEQUENCE ID NO: 1-6. The specific pea proteins from which the peptides are derived are provided in SEQUENCE ID NO: 1-3, and the specific rice proteins from which the peptides are derived are provided in SEQUENCE ID NO: 4-6. Homologs of these proteins are described in SEQUENCE ID NO: 35-53. The specific peptides initially identified in the pea proteins are shown in SEQUENCE ID NO's: 7-25. The specific peptides initially identified in the rice proteins are shown in SEQUENCE ID NO's: 26-35.

In a first aspect, the invention provides an antibacterial peptide, typically 5 to 50 amino acids in length, and comprising an antibacterial fragment of a protein selected from SEQUENCE ID NO's: 1 to 6, or a homolog thereof, or a variant of the antibacterial fragment (hereafter "peptide of the invention").

In one embodiment, the fragment has between 8 and 13 amino acids. In one embodiment, the fragment has a charge of -1 and 0.

Preferably, the c-terminal amino acid is not C, F, I, K, M, P, Q, R, T, V, W

Preferably, the n-terminal amino acid is not C, D, H, K, M, P, Q, T, V, W

Preferably, the c-terminal domain of the fragment does not contain C, M, V or W.

Preferably, the n-terminal domain of the fragment does not contain K, M, R, T, W, C, F, I.

Preferably, the fragment or peptide does not contain C.

Preferably, the fragment or peptide does not contain M.

Preferably, the fragment or peptide does not contain W.

Preferably, the antibacterial fragment is selected from SEQUENCE ID NO's: 7-35, or a antibacterial variant of the fragment.

Preferably, the antibacterial peptide comprises or consists of a fragment selected from SEQUENCE ID NO's: 7-35, or a antibacterial variant of the fragment.

In one embodiment, the anti-bacterial peptide comprises an anti-bacterial fragment of SEQUENCE ID NO: 32, or a variant of the antibacterial fragment.

### [SEQUENCE ID NO: 1 (Pea Protein 1 - P13918)]

Preferably, the peptide comprises an antibacterial fragment of the protein of SEQUENCE ID NO: 1 or a homolog thereof, or an antibacterial variant of the fragment.

Preferably, the peptide comprises an antibacterial fragment selected from SEQUENCE ID NO'S: 7-9, or an antibacterial variant of the fragment.

The invention also provides a composition comprising at least 1, preferably at least 2, preferably at least 3, preferably at least 4, preferably at least 5, preferably at least 6, preferably at least 7, preferably at least 8, preferably at least 9, or preferably at least 10 antibacterial peptides including an antibacterial fragment of the protein of SEQUENCE ID NO: 1 or a homolog thereof, or an antibacterial variant of the fragment. When the composition comprises a plurality of peptides, each comprises a different antibacterial fragment of SEQUENCE ID NO: 1 (for example the fragments of SEQ ID 7-9), or a homolog thereof. Preferably, the composition comprises a first antibacterial peptide comprising a first antibacterial fragment selected from SEQUENCE ID NO: 7-9 (or an antibacterial variant of the fragment), and a second antibacterial peptide comprising a second antibacterial fragment selected from SEQUENCE ID NO: 7-9 (or an antibacterial variant of the fragment). Preferably, the composition comprises substantially all of the fragments of SEQ ID 7-9, or peptides of the invention comprising all of the fragments of SEQ ID 7-9.

Homologs of Pea Protein 1 (SEQUENCE ID NO: 1) include Vicia fabia, Cicer arietinum and Lens culinaris homologs (SEQ ID NO: 53, 36 and 37).

### [SEQUENCE ID NO: 2 (Pea Protein 2 - Q9M3X6)]

Preferably, the peptide comprises an antibacterial fragment of the protein of SEQUENCE ID NO: 2, or a homolog thereof, or an antibacterial variant of the fragment.

Preferably, the peptide comprises an antibacterial fragment selected from SEQUENCE ID NO'S: 10-19, or an antibacterial variant of the fragment.

The invention also provides a composition comprising at least 1, preferably at least 2, preferably at least 3, preferably at least 4, preferably at least 5, preferably at least 6, preferably at least 7, preferably at least 8, preferably at least 9, or preferably at least 10 antibacterial peptides of the invention including an antibacterial fragment of the protein of SEQUENCE ID NO: 2 or a homolog thereof, or an antibacterial variant of the fragment. When the composition comprises a plurality of peptides, each peptide comprises a different antibacterial fragment of SEQUENCE ID NO: 2 (for example the fragments of SEQ ID 10-19), or a homolog thereof. Preferably, the composition comprises a first antibacterial peptide comprising an antibacterial fragment selected from SEQUENCE ID NO 10-19, and a second antibacterial peptide comprising an antibacterial fragment selected from SEQUENCE ID NO 10-19. Preferably, the composition comprises substantially all of the fragments of SEQ ID 10-19, or peptides of the invention comprising all of the fragments of SEQ ID 10-19.

Homologs of Pea Protein 2 (SEQUENCE ID NO: 2) include Pisum abyssinicum, Lathyrus annuus, and Vicia villosa (SEQ ID 38-40).

### [SEQUENCE ID NO: 3 (Pea Protein 3 - D3VNE2)]

Preferably, the peptide comprises an antibacterial fragment of the protein of SEQUENCE ID NO: 3, or a homolog thereof, or an antibacterial variant of the fragment.

Preferably, the peptide comprises an antibacterial fragment selected from SEQUENCE ID NO 20-25, or a antibacterial variant of the fragment.

The invention also provides a composition comprising at least 1, preferably at least 2, preferably at least 3, preferably at least 4, preferably at least 5, preferably at least 6, preferably at least 7, preferably at least 8, preferably at least 9, or preferably at least 10 antibacterial peptides of the invention including an antibacterial fragment of the protein of SEQUENCE ID NO: 3 or a homolog thereof, or an antibacterial variant of the fragment.

Homologs of Pea Protein 3 (SEQUENCE ID NO: 3) include Lens culinaris, Vicia narbonensis, and Medicago truncatula (SEQ ID NO: 41-43).

### [SEQUENCE ID NO: 4 (Rice Protein 1 - P14323)]

Preferably, the peptide comprises an antibacterial fragment of the protein of SEQUENCE ID NO: 4, or a homolog thereof, or an antibacterial variant of the fragment.

Preferably, the peptide comprises an antibacterial fragment selected from SEQUENCE ID NO'S: 26-30, or an antibacterial variant of the fragment.

The invention also provides a composition comprising at least one or more peptides of the invention including an antibacterial fragment of SEQUENCE ID NO: 4 or a homolog thereof. Preferably, the composition comprises a first peptide comprising a antibacterial fragment SEQ ID NO: 26-30 and a second peptide comprising a antibacterial fragment SEQ ID NO: 26-30. Preferably, the composition comprises substantially all of the fragments of SEQ ID 26-30, or peptides of the invention comprising all of the fragments of SEQ ID 26-30.

Homologs of Rice Protein 1 (SEQUENCE ID NO: 4) include Oryza brachyantha, and Zizania latifolia (SEQ ID NO: 44-46).

### [SEQUENCE ID NO: 5 (Rice Protein 2 - P14614)]

Preferably, the peptide comprises an antibacterial fragment of the protein of SEQUENCE ID NO: 5, or a homolog thereof, or an antibacterial variant of the fragment.

Preferably, the peptide comprises an antibacterial fragment selected from SEQUENCE ID NO'S: 31 or 32, or an antibacterial variant of the fragment.

The invention also provides a composition comprising at least 1, preferably at least 2, preferably at least 3, preferably at least 4, preferably at least 5, preferably at least 6, preferably at least 7, preferably at least 8, preferably at least 9, or preferably at least 10 antibacterial peptides of the invention, each of which comprises an antibacterial fragment of SEQUENCE ID NO: 5 or a homolog thereof.

Homologs of Rice Protein 2 (SEQUENCE ID NO: 5) include Oryza sativa Japonica Group and Seed Storage Globulin (SEQ ID 47-49).

### [SEQUENCE ID NO: 6 (Rice Protein 3 - P07728)]

Preferably, the peptide comprises an antibacterial fragment of the protein of SEQUENCE ID NO: 6 or a homolog thereof, or an antibacterial variant of the fragment.

Preferably, the peptide comprises an antibacterial fragment selected from SEQUENCE ID NO'S: 33-35, or an antibacterial variant of the fragment.

The invention also provides a composition comprising at least 1, preferably at least 2, preferably at least 3, preferably at least 4, preferably at least 5, preferably at least 6, preferably at least 7, preferably at least 8, preferably at least 9, or preferably at least 10 antibacterial peptides of the invention, each of which comprises an antibacterial fragment of SEQUENCE ID NO: 6 or a homolog thereof. Preferably, the composition comprises a first antibacterial peptide comprising an antibacterial fragment selected from SEQUENCE ID NO 33-35, and a second antibacterial peptide comprising an antibacterial fragment selected from SEQUENCE ID NO 33-35.

Homologs of Rice Protein 3 (SEQUENCE ID NO: 6) include glutelin (Oryza sativa Japonica Group), Glutelin precursor (zizania latifolia) anmd globulin (avena sativa) (SEQ ID NO: 50-52).

The invention also provides a composition comprising at least one and preferably a plurality of antibacterial peptides of the invention, wherein each of the peptides of the invention comprises an antibacterial fragment of a protein selected from SEQUENCE ID NO: 1 to 6 or a homolog thereof, or an antibacterial variant of the fragment.

Typically, the or each antibacterial peptide of the invention is selected from, or comprises, an antibacterial fragment selected from, SEQUENCE ID NO: 7-35, or an antibacterial variant of the fragment.

The invention also provides a composition comprising at least one and preferably a plurality of peptides of the invention, wherein the or each of the peptides of the invention comprise a antibacterial fragment of a protein selected from SEQUENCE ID NO: 1-3. Typically, the or each peptide of the invention is selected from, or comprises an antibacterial fragment selected from, SEQUENCE ID NO: 7-25, or an antibacterial variant of the fragment. The invention also provides a composition comprising substantially all of the fragments of SEQ ID NO's 7-25, or peptides comprising the fragments, or antibacterial variants of the fragments

The invention also provides a composition comprising at least one and preferably a plurality of peptides of the invention, wherein the or each of the peptides of the invention comprise a antibacterial fragment of a protein selected from SEQUENCE ID NO: 4-6. Typically, the or each peptide of the invention is selected from, or comprises an antibacterial fragment selected from, SEQUENCE ID NO: 26-35, or an antibacterial variant of the fragment. The invention also provides a composition comprising substantially all of the fragments of SEQ ID NO's 26-35, or peptides comprising the fragments, or antibacterial variants of the fragments. Preferably, the composition comprises at least two distinct antibacterial peptides of the invention.

Preferably, the composition comprises at least three distinct antibacterial peptides of the invention.

Preferably, the composition comprises at least four distinct antibacterial peptides of the invention.

Preferably, the composition comprises at least five distinct antibacterial peptides of the invention.

Preferably, the composition comprises at least six distinct antibacterial peptides of the invention.

Preferably, the composition comprises at least seven distinct antibacterial peptides of the invention.

Preferably, the composition comprises at least eight distinct antibacterial peptides of the invention.

Preferably, the composition comprises at least nine distinct antibacterial peptides of the invention.

Preferably, the composition comprises at least ten distinct antibacterial peptides of the invention.

In one embodiment, the invention comprises a composition comprising substantially all of fragments SEQUENCE ID NO: 7-25, or peptides containing the fragments, or antibacterial variants of the fragments, or a mixture of the antibacterial fragments and variants.

In one embodiment, the invention comprises a composition comprising substantially all of fragments SEQUENCE ID NO: 26-35, or peptides containing the fragments, or antibacterial variants of the fragments, or a mixture of the antibacterial fragments and variants.

In one embodiment, the composition comprises a surfactant. In one embodiment the surfactant is selected from a cationic surfactant, an anionic surfactant, a non-ionic surfactant, and a combination thereof. In one embodiment, the composition comprises a fragrance. In one embodiment, the composition includes an anti-perspirant agent. In one embodiment, the composition, the composition includes an oxidising agent (such as a bleach). In one embodiment, the composition is a liquid product. In one embodiment, the composition is a solid product. In one embodiment, the composition is a semi-solid product (for example a cream, lotion or gel). In one embodiment, the solid is a particulate product.

In one embodiment the composition is a personal care composition. Examples of personal care compositions include skincare products, cosmetics, hair cleaning or care products, dentrifices, mouthwashes, anti-perspirants, and deodorants. In one embodiment, the composition is a household cleaning product (to be understood to include industrial cleaning products, hard surface cleaning products, toilet cleaning products, dishwashing liquids, dishwashing tablets, fabric washing tablets). In one embodiment the composition is a plant biocide (i.e. for use in treating plants to kill microbial plant pathogens).

The invention also relates to a comestible product comprising an antibacterial peptide or fragment of the invention. Preferably the comestible product is man-made.

The invention also relates to a comestible product comprising a composition of peptides or fragments of the invention. Preferably the comestible product is man-made.

Preferably, the comestible product is a food product for human or animal (mammalian) or cellular consumption.

In one embodiment the man-made comestible product is a sports nutrition product, for example a beverage, snack or supplement. In one embodiment the man-made comestible product is a beverage. In one embodiment the man-made comestible product is a bakery product. In one embodiment the man-made comestible product is a dairy product. In one embodiment the man-made comestible product is a snack product. In one embodiment the man-made comestible product is a baked extruded food product. In one embodiment the man-made comestible product is powdered milk. In one embodiment the man-made comestible product is an infant formula product. In one embodiment the man-made comestible product is a confectionary product. In one embodiment the man-made comestible product is a yoghurt. In one embodiment the man-made comestible product is a yoghurt drink. In one embodiment the man-made comestible product is an ice cream product. In one embodiment the man-made comestible product is a frozen food product. In one embodiment the man-made comestible product is a breakfast cereal. In one embodiment the man-made comestible product is a bread. In one embodiment the man-made comestible product is a flavoured milk drink. In one embodiment the man-made comestible product is a confectionary bar. In one embodiment the man-made comestible product is a tea or tea product. In one embodiment the man-made comestible product is a based extruded snack product. In one embodiment the man-made comestible product is a fried snack product. In one embodiment the man-made comestible product is a nutritional supplement. In one embodiment the man-made comestible product is a sports nutritional product. In one embodiment the man-made comestible product is a baby food product. In one embodiment the man-made comestible product is a speciality food product for immunocompromised individuals. In one embodiment the man-made comestible product is a food for geriatric patients.

The invention also relates to a peptide or composition of the invention for use in treating or preventing a bacterial infection in a mammal.

The invention also relates to a peptide or composition of the invention for use as an antimicrobial or antibacterial agent.

The invention also relates to a peptide or composition of the invention for use as a preservative.

The invention also relates to a peptide or composition of the invention for use as a preservative in a perishable product, such as a food product or a personal care composition. The invention also relates to a peptide or composition of the invention for use as an anti-bacterial agent in a personal care composition.

The invention also relates to a peptide or composition of the invention for use as an anti-bacterial agent in a household cleaning product.

The invention also relates to a peptide or composition of the invention for use as a plant biocidal agent.

The invention also relates to a peptide of the invention for use in treatment or prevention of a disease or condition characterised by a bacterial infection. The invention also relates to a composition of peptides of the invention for use in treatment or prevention of a disease or condition characterised by a bacterial infection. In one embodiment, the bacterial infection is a MRSA infection.

The invention also relates to a pharmaceutical composition comprising an antibacterial peptide of the invention in combination with a pharmaceutically acceptable carrier.

The invention also relates to a pharmaceutical composition comprising a composition of antibacterial peptides of the invention in combination with a pharmaceutically acceptable carrier.

The invention also relates to a comestible product, for example a food product comprising an antibacterial peptide or composition of the invention, for example a dairy or non-dairy product, a solid food or a beverage, a food additive or supplement. The dairy product may be a milk, a cheese, or yoghurt. In one embodiment, the food product is a sports nutritional product. The food product may comprise any amount of the composition of the invention, for example from 0.1% to 30% (w/w).

The food product may be a Food for Specific Medicinal Purposes (FSMP) which is defined as foods that are specifically formulated, processed and intended for the dietary management of diseases, disorders or medical conditions of individuals who are being treated under medical supervision. These foods are intended for the exclusive or partial feeding of people whose nutritional requirements cannot be met by normal foods.

### Definitions

The term "mammal" should be understood to mean a higher mammal, especially a human or performance mammal such as a dog or horse.

The term "peptide" used herein refers to a polymer composed of 5 to 50 amino acid monomers typically via peptide bond linkage. Peptides (including fragments and variants thereof) of and for use in the invention may be generated wholly or partly by chemical synthesis or by expression from nucleic acid. For example, the peptides of and for use in the present invention can be readily prepared according to well-established, standard liquid or, preferably, solid-phase peptide synthesis methods known in the art (see, for example, J. M. Stewart and J. D. Young, Solid Phase Peptide Synthesis, 2nd edition, Pierce Chemical Company, Rockford, Illinois (1984), in M. Bodanzsky and A. Bodanzsky, The Practice of Peptide Synthesis, Springer Verlag, New York (1984). When necessary, any of the peptides employed in the invention can be chemically modified to increase their stability. A chemically modified peptide or a peptide analog includes any functional chemical equivalent of the peptide characterized by its increased stability and/or efficacy in vivo or in vitro in respect of the practice of the invention. The term peptide analog also refers to any amino acid derivative of a peptide as described herein. A peptide analog can be produced by procedures that include, but are not limited to, modifications to side chains, incorporation of unnatural amino acids and/or their derivatives during peptide synthesis and the use of crosslinkers and other methods that impose conformational constraint on the peptides or their analogs. Examples of side chain modifications include modification of amino groups, such as by reductive alkylation by reaction with an aldehyde followed by reduction with NaBH₄; amidation with methylacetimidate; acetylation with acetic anhydride; carbamylation of amino groups with cyanate; trinitrobenzylation of amino groups with 2, 4, 6, trinitrobenzene sulfonic acid (TNBS); alkylation of amino groups with succinic anhydride and tetrahydrophthalic anhydride; and pyridoxylation of lysine with pyridoxa-5'-phosphate followed by reduction with NABH₄. The guanidino group of arginine residues may be modified by the formation of heterocyclic condensation products with reagents such as 2,3-butanedione, phenylglyoxal and glyoxal. The carboxyl group may be modified by carbodiimide activation via o-acylisourea formation followed by subsequent derivatization, for example, to a corresponding amide. Sulfhydryl groups may be modified by methods, such as carboxymethylation with iodoacetic acid or iodoacetamide; performic acid oxidation to cysteic acid; formation of mixed disulphides with other thiol compounds; reaction with maleimide; maleic anhydride or other substituted maleimide; formation of mercurial derivatives using 4-chloromercuribenzoate, 4-chloromercuriphenylsulfonic acid, phenylmercmy chloride, 2-chloromercuric-4-nitrophenol and other mercurials; carbamylation with cyanate at alkaline pH. Tryptophan residues may be modified by, for example, oxidation with N-bromosuccinimide or alkylation of the indole ring with 2-hydroxy-5-nitrobenzyl bromide or sulphonyl halides. Tryosine residues may be altered by nitration with tetranitromethane to form a 3-nitrotyrosine derivative. Modification of the imidazole ring of a histidine residue may be accomplished by alkylation with iodoacetic acid derivatives or N-carbethoxylation with diethylpyrocarbonate. Examples of incorporating unnatural amino acids and derivatives during peptide synthesis include, but are not limited to, use of norleucine, 4-amino butyric acid, 4-amino-3-hydroxy-5-phenylpentanoic acid, 6-aminohexanoic acid, t-butylglycine, norvaline, phenylglycine, ornithine, sarcosine, 4-amino-3-hydroxy-6-methylheptanoic acid, 2-thienyl alanine and/or D-isomers of amino acids. Peptide structure modification includes the generation of retro-inverso peptides comprising the reversed sequence encoded by D-amino acids.

"Fragment" means a segment of a protein selected from SEQUENCE ID NO's: 1 to 6. Typically the fragment is 9-12 contiguous amino acids in length. Generally, the fragment has a charge of between -1 and 0. The charge of a peptide, fragment or region is determined using the method of Cameselle, J.C., Ribeiro, J.M., and Sillero, A. (1986). Derivation and use of a formula to calculate the net charge of acid-base compounds. Its application to amino acids, proteins and nucleotides. Biochem. Educ. 14, 131-136.

The term "natural" as applied to an antibacterial peptide means an antibacterial peptide that includes (a) an antibacterial fragment of a plant protein, typically rice or pea protein, or variants of pea protein including lentil, sweet pea, or chick pea or variants of rice protein including oat, grass, corn, wild rice and bananas, or (b) an antibacterial variant of the fragment of a plant protein, for example an antibacterial fragment of a homolog of the plant protein. The peptides or fragments of the invention may be isolated from plant protein hydrolysates or made synthetically using methods known to a person skilled in the art and described herein.

"C-terminal domain" as applied to a fragment means the first three amino acids at the c-terminus of the fragment.

"N-terminal domain" as applied to a fragment means the last three amino acids at the n-terminus of the fragment.

"Antibacterial" or "antibacterial activity" as applied to a peptide or fragment means a peptide or fragment that is capable of visibly inhibiting the growth of a bacteria in the agar-plate based growth inhibition studies described below.

"Homolog" of a reference protein should be understood to mean a protein from a different species of plant having at least 60%, 70%, 80%, 90%, 91%, 92%, 93%, 94%, 95%, 96%, 97%, 98%, 99% sequence homology with the reference protein. Thus, for example, homologs of pea protein P13918 include:
>gi|137584|sp|P08438.1|VCL_V|CFARecName:Full=Vicilin; Flags: Precursor [Vicia faba]
>gi|22057|emb|CAA68559.1|vicilin [Vicia faba var. minor]>gi|383931031|gb|AFH56916.1| vicilin **[Vicia faba]**
>gi||502105533|ref|XP-004492829.1|PREDICTED: vicilin-like isoform X1 **[Cicer arietinum] ChickPea**
>gi|29539109|emb|CAD87730.1|allergen Len c 1.0101 **[Lens culinaris] Lentil**

A "variant" of an antibacterial fragment shall be taken to mean a fragment having an amino acid sequence that is substantially identical to the reference antibacterial fragment, and which has antibacterial activity as defined above. Thus, for example, the term should be taken to include fragments that are altered in respect of one or more amino acid residues. Preferably such alterations involve the insertion, addition, deletion and/or substitution of 5 or fewer amino acids, more preferably of 4 or fewer, even more preferably of 3 or fewer, most preferably of 1 or 2 amino acids only. Insertion, addition and substitution with natural and modified amino acids is envisaged. The variant may have conservative amino acid changes, wherein the amino acid being introduced is similar structurally, chemically, or functionally to that being substituted. Generally, the variant will have at least 70% amino acid sequence homology, preferably at least 80% sequence homology, more preferably at least 90% sequence homology, and ideally at least 95%, 96%, 97%, 98% or 99% sequence homology with the reference antibacterial fragment. In this specification, the term "sequence homology" should be understood to comprise both sequence identity and similarity, i.e. a variant (or homolog) that shares 70% sequence homology with a reference sequence is one in which any 70% of aligned residues of the variant (or homolog) are either identical to, or conservative substitutions of, the corresponding residues in the reference sequence.

"Consisting essentially of" as applied to a plurality of peptides means substantially all of the peptides, for example at least 60%, 70%, 80% or 90% of the peptides.

"Pharmaceutical compositions": A further aspect of the invention relates to a pharmaceutical composition comprising a peptide of the invention or a composition of peptides of the invention, admixed with one or more pharmaceutically acceptable diluents, excipients or carriers. Even though the peptides and compositions of the present invention can be administered alone, they will generally be administered in admixture with a pharmaceutical carrier, excipient or diluent, particularly for human therapy. The pharmaceutical compositions may be for human or animal usage in human and veterinary medicine. Examples of such suitable excipients for the various different forms of pharmaceutical compositions described herein may be found in the "Handbook of Pharmaceutical Excipients, 2nd Edition, (1994), Edited by A Wade and PJ Weller. In particular, formulations for topical delivery are described in Topical drug delivery formulations edited by David Osborne and Antonio Aman, Taylor & Francis, the complete contents of which are incorporated herein by reference. Acceptable carriers or diluents for therapeutic use are well known in the pharmaceutical art, and are described, for example, in Remington's Pharmaceutical Sciences, Mack Publishing Co. (A. R. Gennaro edit. 1985). Examples of suitable carriers include lactose, starch, glucose, methyl cellulose, magnesium stearate, mannitol, sorbitol and the like. Examples of suitable diluents include ethanol, glycerol and water. The choice of pharmaceutical carrier, excipient or diluent can be selected with regard to the intended route of administration and standard pharmaceutical practice. The pharmaceutical compositions may comprise as, or in addition to, the carrier, excipient or diluent any suitable binder(s), lubricant(s), suspending agent(s), coating agent(s), solubilising agent(s). Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol. Examples of suitable lubricants include sodium oleate, sodium stearate, magnesium stearate, sodium benzoate, sodium acetate, sodium chloride and the like. Preservatives, stabilizers, dyes and even flavouring agents may be provided in the pharmaceutical composition. Examples of preservatives include sodium benzoate, sorbic acid and esters of p-hydroxybenzoic acid. Antioxidants and suspending agents may be also used.

The peptide or composition of the invention may be adapted for topical, oral, rectal, parenteral, intramuscular, intraperitoneal, intra-arterial, intrabronchial, subcutaneous, intradermal, intravenous, nasal, vaginal, buccal or sublingual routes of administration. For oral administration, particular use is made of compressed tablets, pills, tablets, gellules, drops, and capsules. Preferably, these compositions contain from 1 to 250 mg and more preferably from 10-100 mg, of active ingredient per dose. Other forms of administration comprise solutions or emulsions which may be injected intravenously, intra-arterial, subcutaneously, intradermally, intraperitoneally or intramuscularly, and which are prepared from sterile or sterilisable solutions. The pharmaceutical compositions of the present invention may also be in form of suppositories, vaginal rings, pessaries, suspensions, emulsions, lotions, ointments, creams, gels, sprays, solutions or dusting powders. The composition of the invention may be formulated for topical delivery. Topical delivery generally means delivery to the skin, but can also mean delivery to a body lumen lined with epithelial cells, for example the lungs or airways, the gastrointestinal tract, the buccal cavity. In particular, formulations for topical delivery are described in Topical drug delivery formulations edited by David Osborne and Antonio Aman, Taylor & Francis, the complete contents of which are incorporated herein by reference. Compositions or formulations for delivery to the airways are described in O'Riordan et al (Respir Care, 2002, Nov. 47), EP2050437, WO2005023290, US2010098660, and US20070053845. Composition and formulations for delivering active agents to the iluem, especially the proximal iluem, include microparticles and microencapsulates where the active agent is encapsulated within a protecting matrix formed of polymer or dairy protein that is acid resistant but prone to dissolution in the more alkaline environment of the ileum. Examples of such delivery systems are described in EP1072600.2 and EP13171757.1. An alternative means of transdermal administration is by use of a skin patch. For example, the active ingredient can be incorporated into a cream consisting of an aqueous emulsion of polyethylene glycols or liquid paraffin. The active ingredient can also be incorporated, at a concentration of between 1 and 10% by weight, into an ointment consisting of a white wax or white soft paraffin base together with such stabilisers and preservatives as may be required.
Injectable forms may contain between 10-1000 mg, preferably between 10-250 mg, of active ingredient per dose.

Compositions may be formulated in unit dosage form, i.e., in the form of discrete portions containing a unit dose, or a multiple or sub-unit of a unit dose.

A person of ordinary skill in the art can easily determine an appropriate dose of one of the instant compositions to administer to a subject without undue experimentation. Typically, a physician will determine the actual dosage which will be most suitable for an individual patient and it will depend on a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy. The dosages disclosed herein are exemplary of the average case. There can of course be individual instances where higher or lower dosage ranges are merited, and such are within the scope of this invention. Depending upon the need, the agent may be administered at a dose of from 0.01 to 30 mg/kg body weight, such as from 0.1 to 10 mg/kg, more preferably from 0.1 to 1 mg/kg body weight. In an exemplary embodiment, one or more doses of 10 to 300 mg/day or more preferably, 10 to 150 mg/day, will be administered to the patient for the treatment of an inflammatory disorder.

In a particularly preferred embodiment, the methods and uses of the invention involve administration of a peptide or composition of the invention in combination with one or more other active agents, for example, existing antibacterial drugs or pharmacological enhancers available on the market. In such cases, the compounds of the invention may be administered consecutively, simultaneously or sequentially with the one or more other active agents.

In one embodiment of the invention, the peptide of the invention may be administered in the form of a conjugate comprising the peptide, a linker, and an antibody molecule intended to increase the half-life of the conjugate in-vivo.

"Man-made" as applied to comestible products should be understood to mean made by a human being and not existing in nature.

"Disease or condition characterised by bacterial infection" means any condition or disease characterised having a pathology caused by growth of bacteria or by bacterial infection, including for example MRSA, salmonella, listeria, bacterial pneumonia, Staphylococcal food poisoning, bacterial memingitis. Specific examples are provided in https://en.wikipedia.org/wiki/List_of_infectious_diseases.

In the specification the terms "comprise, comprises, comprised and comprising" or any variation thereof and the terms "include, includes, included and including" or any variation thereof are considered to be totally interchangeable and they should all be afforded the widest possible interpretation and vice versa.

### Brief Description of the Figures

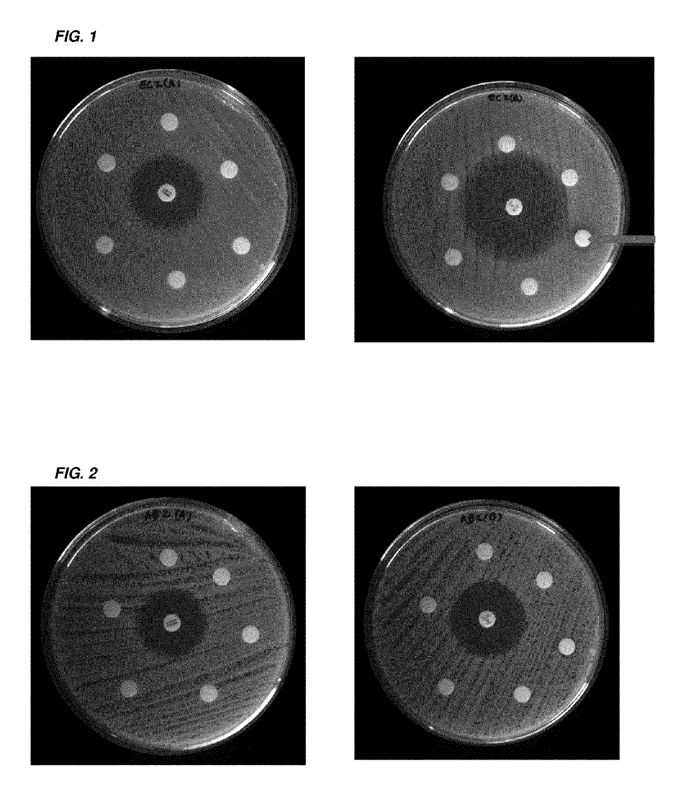

Figs 1A and 1B are pictures of agar plates in which *E Coli* ATCC25922 is growing showing an inhibition zone (9-10mm) obtained with peptide I_87_SF (arrow). Control antibiotic discs (TET and CIP) were placed in the centre of each plate.
Figs 2A and 2B are pictures of agar plates in which *Acinetobacter baumannii* 19606 is growing, No antibacterial activity was detected. Control antibiotic discs (TET and CIP) were placed in the centre of each plate.

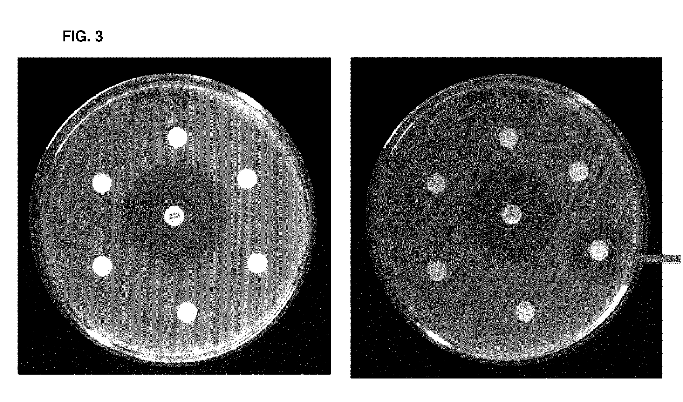

Figs 3A and 3B are pictures of agar plates in which MRSA 4330 is growing showing an inhibition zone (18mm) obtained with peptide I_87_SF (arrow). Control antibiotic discs (TET and CIP) were placed in the centre of each plate.

### Detailed Description of the Invention

The following peptides were tested for bacterial inhibition activity in solid and liquid media test. I_87_SF [SEQ ID 32] is an antibacterial fragment of Rice Protein P14614, whereas the remaining six peptides are comparative peptides.
- I_45_SF: ITSVNSQKFPILNLIQMSATR
- I_77_SF: SRVQVVSNFGK
- E_31_SF: VLDLAIPVNKPGQLQSFLLSGTQNQPSLLSGFSK
- E_376_SF: NAMFVPHYNLNANSIIYALKGR
- E_78_SF: LRPGVMFVVPAGHPFVNIASK
- E_354_SF: GLFDLGHPLVNR
- I_87_SF: LNSQKFPILNLVQLSATR [SEQ ID 32]

### UCD_CFS_Strains used for Determination of antibacterial activity

| | **Strain** | **Type** | **Hospital/Isolation** | **Source** | **ARP (Resistant to)** | **Reference** |
|---|---|---|---|---|---|---|
| **Gram-negative** | *E. coli* 25922 | Reference | FDA strain Seattle 1946 [DSM 1103, NCIB 12210] | - | PEN; VAN; AMP; CLI; CL | http://www.atcc.org/ATCCAdvancedCatalogSea rch/ProductDetails/tabid/452/Default.aspx?ATC CNum=25922&Template=bacteria |
| | *Acinetobacter baumannii* ATCC 19606 | Reference | Deposit in ATCC as *Bacterium anitratum* Schaub and Hauber 2208 [81, DSM 6974] | Urine | - | Hugh R, Reese R. Designation of the type strain for Bacterium anitratum Schaub and Hauber 1948. Int. J. Syst. Bacteriol. 17: 245-254, 1967. |
| **Gram-positive** | MRSA ATCC 43300 | Reference | Kansas | Human | AMP; PEN; OXA; MET; AXO; CIP; LEVO; GAT; ERY; CLI | http://www.atcc.org/ATCCAdvancedCatalogSea rch/ProductDetails/tabid/452/Default.aspx?ATC CNum=43300&Template=bacteria |

| | | | | | | |
|---|---|---|---|---|---|---|
| **Abbreviations:** MRSA - Methicillin-Resistant *Staphylococcus aureus*; ARP - Antibiotic Resistance Profile; AMC - Amoxicillin-Clavulanic acid; C-Chloramphenicol; F- Furazolidone; Fc- Florfenicol Gm-Gentamycin; N-Neomycin; NAL-Nalidixic acid; S-Streptomycin; Su-Sulfonamides; TET-Tetracycline; TMP-Trimetoprim; AMP-ampicillin; PEN - penicillin; OXA - oxacillin; MET-methicillin; AXO - ceftriaxone; CIP-ciprofloxacin; LEVO-levofloxacin; GAT - gatifloxacin; ERY-erythromycin; CLI- clindamycin; CL- Cephalexin | | | | | | |

**Compounds tested.** A total of 1 compound (1 peptide) was tested. Peptide stock=5mg/mL dissolved in DMSO.

**Preparation of the peptide.** The powder was reconstituted with 1.04 mL of DMSO to achieve a final concentration of 5mg/mL. The peptide was in high purity. No precipitation problems.

**Antibacterial activity testing (in solid media).** Bacterial inoculums were adjusted to McFarland 0.5 standard and MHA plates swabbed. Blank disks were placed in the plates and 10 µL of each compound (at 64 µg/mL - maximum concentration tested) added. Plates were incubated at 37°C for 16-18 hours. Appropriate controls (DMSO; Mueller-Hinton media alone; and two antibiotic discs - ciprofloxacin and tetracycline) were also performed.

### Results from the testing in agar-plates

Determination of antibacterial activity (inhibition of growth) was performed in Mueller-Hinton plates. After incubation period results were registered and plates photographed.

| **Compounds** | **Inhibition zone diameter (mm)** | | | | | | | | |
|---|---|---|---|---|---|---|---|---|---|
| **ID** | ***E. coli* ATCC 25922** | | | ***A. baumannii* ATCC 19606** | | | **MRSA ATCC 43300** | | |
| | **Rep 1** | **Rep 2** | **Rep 3** | **Rep 1** | **Rep 2** | **Rep 3** | **Rep 1** | **Rep 2** | **Rep 3** |
| **I_87_SF** | 10 | 10 | 9 | NI | NI | NI | 18 | 18 | 18 |
| **TET** | **28** | **26** | **26** | **23** | **24** | **24** | **31** | **31** | **32** |
| **CIP** | **42** | **40** | **42** | **27** | **28** | **27** | **27** | **28** | **29** |
| **DMSO** | NI | NI | NI | NI | NI | NI | NI | NI | NI |
| **MH** | NI | NI | NI | NI | NI | NI | NI | NI | NI |

| | | | | | | | | | |
|---|---|---|---|---|---|---|---|---|---|
| **Legend:** TET - tetracycline; CIP - Ciprofloxacin; MH - Mueller-Hinton (control); NI - No Inhibition of growth | | | | | | | | | |

**Final result:** Peptide I_87_SF showed some inhibitory activity against *E. coli* and MRSA, but not at the levels of susceptibility. No activity was obtained against *A. baumannii*.

### Results from the testing in agar-plates (photos)

**Note:** Only one set is shown since the other two sets had the same results. Control antibiotic discs (TET and CIP) were placed in the centre of each plate.

The invention is not limited to the embodiments hereinbefore described which may be varied in construction and detail without departing from the spirit of the invention.

### SEQUENCES

PROTEIN : P13918-2-Pea

MAATTMKASFPLLMLMGISFLASVCVSSRSDPQNPFIFKSNKFQTLFENENGHIRLLQKFDQRSKIFENLQNYRLLEYKSKPHTIFLPQHTDADYI
LVVLSGKAILTVLKPDDRNSFNLERGDTIKLPAGTIAYLVNRDDNEELRVLDLAIPVNRPGQLQSFLLSGNQNQQNYLSGFSKNILEASFNTDYEE
IEKVLLEEHEKETQHRRSLKDKRQQSQEENVIVKLSRGQIEELSKNAKSTSKKSVSSESEPFNLRSRGPIYSNEFGKFFEITPEKNPQLQDLDIFVNS
VEIKEGSLLLPHYNSRAIVIVTVNEGKGDFELVGQRNENQQEQRKEDDEEEEQGEEEINKQVQNYKAKLSSGDVFVIPAGHPVAVKASSNLDLL
GFGINAENNQRNFLAGDEDNVISQIQRPVKELAFPGSAQEVDRILENQKQSHFADAQPQQRERGSRETRDRLSSV [SEQ ID 1]

   PEPTIDE : GSLLLPHYN [SEQ ID 7]
   PEPTIDE : GSLLLPHYNS [SEQ ID 8]
   PEPTIDE : SSNLDLLGFG [SEQ ID 9]
PROTEIN : Q9M3X6 - 3 - Pea

MATTIKSRFPLLLLLGIIFLASVVCVTYANYDEGSEPRVPAQRERGRQEGEKEEKRHGEWRPSYEKEEDEEEGQRERGRQEGEKEEKRHGEWR
PSYEKQEDEEEKQKYRYQREKEDEEEKQKYQYQREKKEQKEVQPGRERWEREEDEEQVDEEWRGSQRREDPEERARLRHREERTKRDRRHQ
REGEEEEERSSESQERRNPFLFKSNKFLTLFENENGHIRLLQRFDKRSDLFENLQNYRLVEYRAKPHTIFLPQHIDADLILVVLSGKAILTVLSPNDR
NSYNLERGDTIKLPAGTTSYLVNQDDEEDLRLVDLVIPVNGPGKFEAFDLAKNKNQYLRGFSKNILEASYNTRYETIEKVLLEEQEKDRKRRQQG
EETDAIVKVSREQIEELKKLAKSSSKKSLPSEFEPINLRSHKPEYSNKFGKLFEITPEKKYPQLQDLDLFVSCVEINEGALMLPHYNSRAIVVLLVNEG
KGNLELLGLKNEQQEREDRKERNNEVQRYEARLSPGDVVIIPAGHPVAITASSNLNLLGFGINAENNERNFLSGSDDNVISQIENPVKELTFPGS
VQEINRLIKNQKQSHFANAEPEQKEQGSQGKRSPLSSILGTFY [SEQ ID 2]

   PEPTIDE : ELLGLKNE [SEQ ID 10]
   PEPTIDE : SNLNLLGFG [SEQ ID 11]
   PEPTIDE : ASSNLNLL [SEQ ID 12]
   PEPTIDE : YPQLQDLDL [SEQ ID 13]
   PEPTIDE : SSNLNLLGFG [SEQ ID 14]
   PEPTIDE : ASSNLNLLG [SEQ ID 15]
   PEPTIDE : LLGLKNEQQE [SEQ ID 16]
   PEPTIDE : LVVLSGKAIL [SEQ ID 17]
   PEPTIDE : LNLLGFGI [SEQ ID 18]
   PEPTIDE : ASSNLNLLGF [SEQ ID 19]
PROTEIN : D3VNE2 - 5 - Pea

MAATPIKPLMLLAIAFLASVCVSSRSDQENPFIFKSNRFQTLYENENGHIRLLQKFDKRSKIFENLQNYRLLEYKSKPHTLFLPQYTDADFILVVLN
GKATLTVLKSNDRNSFNLERGDTIKLPAGTIAYLANRDDNEDLRVLDLAIPVNKPGQLQSFLLSGTQNQPSLLSGFSKNILEAAFNTNYEEIEKVL
LEQQEQEPQHRRSLKDRRQEINEENVIVKVSREQIEELSKNAKSSSKKSVSSESGPFNLRSRNPIYSNKFGKFFEITPEKNQQLQDLDIFVNSVDIK
EGSLLLPNYNSRAIVIVTVTEGKGDFELVGQRNENQGKENDKEEEQEEETSKQVQLYKAKLSPGDVFVIPAGHPVAINASSDLNLIGFGINAEN
NERNFLAGEEDNVISQVQRPVKELAFPGSSHEIDRLLKNQKQSYFANAQPLQRE [SEQ ID 3]

   PEPTIDE : LQNYRLLE [SEQ ID 20]
   PEPTIDE : LLSGTQNQPSLL [SEQ ID 21]
   PEPTIDE : LLLPNYNSR [SEQ ID 22]
   PEPTIDE : NLQNYRLLE [SEQ ID 23]
   PEPTIDE : GSLLLPNYNS [SEQ ID 24]
   PEPTIDE : NLQNYRLL [SEQ ID 25]
PROTEIN : P14323 - 2 - Rice

MASSVFSRFSIYFCVLLLCHGSMAQLFNPSTNPWHSPRQGSFRECRFDRLQAFEPLRKVRSEAGVTEYFDEKNELFQCTGTFVIRRVIQPQGLL
VPRYTNIPGVVYIIQGRGSMGLTFPGCPATYQQQFQQFSSQGQSQSQKFRDEHQKIHQFRQGDIVALPAGVAHWFYNDGDAPIVAVYVYDV
NNNANQLEPRQKEFLLAGNNNRAQQQQVYGSSIEQHSGQNIFSGFGVEMLSEALGINAVAAKRLQSQNDQRGEIIHVKNGLQLLKPTLTQQ
QEQAQAQDQYQQVQYSERQQTSSRWNGLEENFCTIKVRVNIENPSRADSYNPRAGRITSVNSQKFPILNLIQMSATRVNLYQNAILSPFWNV
NAHSLVYMIQGRSRVQVVSNFGKTVFDGVLRPGQLLIIPQHYAVLKKAEREGCQYIAIKTNANAFVSHLAGKNSVFRALPVDVVANAYRISRE
QARSLKNNRGEEHGAFTPRFQQQYYPGLSNESESETSE [SEQ ID 4]

   PEPTIDE : NGLQLLKPTL [SEQ ID 26]
   PEPTIDE : GLQLLKPTL [SEQ ID 27]
   PEPTIDE : GVLRPGQLL [SEQ ID 28]
   PEPTIDE : DGVLRPGQLL [SEQ ID 29]
   PEPTIDE : LQLLKPTLTQQQE [SEQ ID 30]
PROTEIN : P14614-4-Rice

MATIAFSRLSIYFCVLLLCHGSMAQLFGPNVNPWHNPRQGGFRECRFDRLQAFEPLRRVRSEAGVTEYFDEKNEQFQCTGTFVIRRVIEPQGL
LVPRYSNTPGMVYIIQGRGSMGLTFPGCPATYQQQFQQFLPEGQSQSQKFRDEHQKIHQFRQGDIVALPAGVAHWFYNEGDAPVVALYVF
DLNNNANQLEPRQKEFLLAGNNNREQQMYGRSIEQHSGQNIFSGFNNELLSEALGVNALVAKRLQGQNDQRGEIIRVKNGLKLLRPAFAQQ
QEQAQQQEQAQAQYQVQYSEEQQPSTRCNGLDENFCTIKARLNIENPSHADTYNPRAGRITRLNSQKFPILNLVQLSATRVNLYQNAILSPF
WNVNAHSLVYIVQGHARVQVVSNLGKTVFNGVLRPGQLLIIPQHYVVLKKAEHEGCQYISFKTNANSMVSHLAGKNSIFRAMPVDVIANAYR
ISREQARSLKNNRGEELGAFTPRYQQQTYPGFSNESENEALE [SEQ ID 5]

   PEPTIDE : LSEALGVNAL [SEQ ID 31]
   PEPTIDE : **LNSQKFPILNLVQLSATR** [SEQ ID 32]
PROTEI N : P07728 - 2 - Rice

MASINRPIVFFTVCLFLLCNGSLAQQLLGQSTSQWQSSRRGSPRECRFDRLQAFEPIRSVRSQAGTTEFFDVSNEQFQCTGVSVVRRVIEPRGL
LLPHYTNGASLVYIIQGRGITGPTFPGCPESYQQQFQQSGQAQLTESQSQSQKFKDEHQKIHRFRQGDVIALPAGVAHWCYNDGEVPVVAIY
VTDLNNGANQLDPRQRDFLLAGNKRNPQAYRREVEERSQNIFSGFSTELLSEALGVSSQVARQLQCQNDQRGEIVRVEHGLSLLQPYASLQE
QEQGQVQSRERYQEGQYQQSQYGSGCSNGLDETFCTLRVRQNIDNPNRADTYNPRAGRVTNLNTQNFPILSLVQMSAVKVNLYQNALLSP
FWNINAHSVVYITQGRARVQVVNNNGKTVFNGELRRGQLLIIPQHYAVVKKAQREGCAYIAFKTNPNSMVSHIAGKSSIFRALPNDVLANAY
RISREEAQRLKHNRGDEFGAFTPIQYKSYQDVYNAAESS [SEQ ID 6]

   PEPTIDE : HGLSLLQPYA [SEQ ID 33]
   PEPTIDE : HGLSLLQPYASL [SEQ ID 34]
   PEPTIDE : HGLSLLQPY [SEQ ID 35]

### Examples of homologs for each protein

P13918(Pea)
   >gi|137584|sp|P08438.1|VCL_VICFA RecName: Full=Vicilin; Flags: Precursor [Vicia faba] >gi|22057|emb|CAA68559.1| vicilin [Vicia faba var. minor] >gi| 383931031|gb|AFH56916.1| vicilin **[Vicia faba]** 
MAATTLKDSFPLLTLLGIAFLASVCLSSRSDQDNPFVFESNRFQTLFENENGHIRLLQKFDQHSKLLENLQNYRLLEYKSKPHTIFLPQQTDADFIL
VVLSGKAILTVLLPNDRNSFSLERGDTIKLPAGTIGYLVNRDDEEDLRVLDLVIPVNRPGEPQSFLLSGNQNQPSILSGFSKNILEASFNTDYKEIEK
VLLEEHGKEKYHRRGLKDRRQRGQEENVIVKISRKQIEELNKNAKSSSKKSTSSESEPFNLRSREPIYSNKFGKFFEITPKRNPQLQDLNIFVNYVEI
NEGSLLLPHYNSRAIVIVTVNEGKGDFELVGQRNENQQGLREEYDEEKEQGEEEIRKQVQNYKAKLSPGDVLVIPAGYPVAIKASSNLNLVGFGI
NAENNQRYFLAGEEDNVISQIHKPVKELAFPGSAQEVDTLLENQKQSHFANAQPRERERGSQEIKDHLYSILGSF [SEQ ID 53]
   >gi|502105533|ref|XP_004492829.1| PREDICTED: vicilin-like isoform X1 **[Cicer arietinum] ChickPea** 
MAIKARFPLLVLLGIVFLASVCAKSDKENPFFFKSNNCQTLFENENGHVRLLQRFDKRSQLFENLQNYRLMEYNSKPHTLFLPQHNDADFILVVL
RGRAILTVLNPNDRNTFKLERGDTIKLPAGTIAYLANRDDNEDLRVLDLAIPVNRPGQFQSFSLSGNENQQSYFQGFSKKILEASFNSDYEEIERV
LLEEQEQKPEQRRGHKGRQQSQETDVIVKISREQIEELSKNAKSNCKKSVSSESEPFNLRSRSPIYSNRFGNFFEITPEKNPQLKDLDIFVNSVEIK
EGSLLLPHFNSRATVILVVNEGKGEVELVGLRNENEQENKKEDEEEEEDRNVQVQRFQSKLSSGDVVVIPASHPFSINASSDLFLLGFGINAQN
NQRNFLAGEEDNVISQIQRPVKEVAFPGSAEEVDRLLKNQRQSHFANAQPQQKRKGSQRIRSPF [SEQ ID 36]
   >gi|29539109|emb|CAD87730.1| allergen Len c 1.0101 **[Lens culinaris] Lentil** 
SRSDQENPFIFKSNRFQTIYENENGHIRLLQRFDKRSKIFENLQNYRLLEYKSKPHTIFLPQFTDADFILVVLSGKAILTVLNSNDRNSFNLERGDTI
KLPAGTIAYLANRDDNEDLRVLDLAIPVNRPGQLQSFLLSGTQNQPSFLSGFSKNILEAAFNTEYEEIEKVLLEEQEQKSQHRRSLRDKRQEITNE
DVIVKVSREQIEELSKNAKSSSKKSVSSESEPFNLRSRNPIYSNKFGKFFEITPEKNPQLQDLDIFVNSVEIKEGSLLLPNYNSRAIVIVTVNEGKGDF
ELVGQRNENQQEQREENDEEEGQEEETTKQVQRYRARLSPGDVLVIPAGHPVAINASSDLNLIGFGINAKNNQRNFLAGEEDNVISQIQRPV
KELAFPGSSREVDRLLTNQKQSHFANAQPLQIE [SEQ ID 37]
Q9M3X6 (Pea)
   >gi|164512526|emb|CAP06312.1| cvc [Pisum abyssinicum] 
MATTVESRFPLLLFPGIIFLASVCVTYANYDEGSETRVPGQRERGRQEGEKEEKRHGEWRPSYEKEEDEEEKQKYRYQREKEDEEEKQKYRYQR
EKKEEKEVQPGRERWEREEDEEQVDEEWRGSQRRQDPEERARLRHREERTKRDRRHKREGEEEERSSESQEQRNPFLFKSNKFLTLFENENG
HIRRLQRFDKRSDLFENLQNYRLVEYRAKPHTIFLPQHIDADLILVVLNGKAILTVLSPNDRNSYNLERGDTIKIPAGTTSYLVNQDDEEDLRVVD
FVIPVNRPGKFEAFGLSENKNQYLRGFSKNILEASLNTKYETIEKVLLEEQEKKPQQLRDRKRRQQGGERDAIIKVSREQIEELRKLAKSSSKKSLPS
EFEPFNLRSHKPEYSNKFGKLFEITPEKKYPQLQDLDILVSCVEINKGALMLPHYNSRAIVVLLVNEGKGNLELLGLKNEQQEREDRKERNNEVQ
RYEARLSPGDVVIIPAGHPVAISASSNLNLLGFGTNAENNQRNFLSGSDDN [SEQ ID 38]
   >gi|164512538|emb|CAP06318.1| cvc [Lathyrus annuus] 
MATTIKSRFPLLLLLGIIFLASVCVTWANYDEGSEPRVPGQRERGRQEGEKEEKRHGEWRPSYEEEYDEGLEPKVPGKRERGRQEGEKEEKRHE
EWRPSYEKEEDEEEKQKYNYQREKKEHKEVQPGRERWERKQDEKQVEEDEEPGEEQWRGSKRHEDPEERARLRHREEKTKSYVEDNEETSS
KEGRNPFLFKSNKFLTLFENENGHIRRLQRFDERSDIFENLQNYRLVEYRAKPHTMFLPQHIDADLILVVLNGKAILTVLSPNDRNSYNLERGDT
VKLPAGTTSYLVNQDDEEDLRVVDLAIPVNRPGKFEAFGLSANKNQYLRGFSKNILEASLNTKYETIEKVLLEERRDQKGRQQGQETNAIVKVSR
EQIEELRKLAKSSSKKSLLSESEPLNLRSQNPKYSNKFGKFFEITPQKKYPQLQDLDVSISCVEINKGALLLPHYNSRSIGILLVNEGKGNLELVGFKN
EQQRQRENEETNKKLQRYEARLSSGDVVVIPEGHPVAISASSNLNLLGFGINAANNQRNFLTGSDDN [SEQ ID 39]
   >gi|164512558|emb|CAP06328.1| cvc [Vicia villosa] 
MATTIKSRFPVLLLLGIIFLTSVCVTYANYDEGREPSVPGQRERGRQEGEKEEKRHGEWRPSEEDEEEKYKYEEGRVPGQRERGRQEGEKEEKR
HGKWRPSEEEDEEEKYRYEEGSEPRGPGQRETGRQEGEKEKQRPEREPSYEKEEDEEEKQKYQYHREKKEQREVRPGRERFERHEDEEQWRG
IQRHEDPEERARERYRAEIAKRQVEEEREERDIPHEREQRNPFLFKSNKFQTLFQNENGYIRRLQRFDKRSDLFENLQNYRLVEYRAKPHTIFLPQ
HIDADLIIVVLSGRAILTVLSPDDRNSYNLERGDTIKLPAGTTSYLVNQDDEEDLRVVDLAIPVNRPGKVESFLLSGNKNQYLRGFSKNILEASFNT
NYETIERVLLEEQDKESQQSIGQKRRSQRQETNALVKVSREQLEDLKRLAKSSSQEGLSSQFEPINLRSQNPKYSNKFGKVFEITPEKKYPQLQDL
DLFVSSVDIKEGALMLPHYNSRAIVVLLVNEGRGNLELVGLKNEQQEQREKEDEQQERNNQVQRYEARLSPGDVVIIPAGHPVAVRASSDLNL
LAFGINAENNQRNFLAGSDDN [SEQ ID 40]
D3VNE2 (Pea)
   >gi|29539111|emb|CAD87731.1| allergen Len c 1.0102 [Lens culinaris] 
SRSDQENPFIFKSNRFQTIYENENGHIRLLQKFDKRSKIFENLQNYRLLEYKSKPHTLFLPQYTDADFILVVLSGKAVLTVLNSNDRNSFNLERGDT
IKLPAGTIAYLANRDDNEDLRVLDLAIPVNNPGQLESFLLSGTQNQPSFLSGFNKSILEAAFNTDYEEIEKVLLEDQEQEPQHRRSLRDRRQEINK
ENVIVKVSREQIKELSKNAKSSSKKSVSSESEPFNLRSRNPIYSNKFGKFFEITPEKNPQLQDLDIFVNSVEIKEGSLLLPNYNSRAIVIVTVNEGKGY
FELVGQRNENQREENDDEEEQEEETSTQVQRYRAKLSPGDVFVVPAGHPVAINASSDLNLIGFGINAKNNQRNFLAGEEDNVISQIQRPVKEL
AFPGSSREVDRLLTNQKQSHFANAQPLQIE [SEQ ID 41]
   >gi|1297072|emb|CAA96514.1| vicilin precursor [Vicia narbonensis] 
MAAITMKVSFPLLMLLGISFLASVCVSSRSDQENPFIFKSNKFQTLFENDNGHIRLLQKFDERSKILENLQNYRLLEYKSKPRTIFLPQQTNADFIL
VVLSGKAILTVLKPDDRNSFNLERGDTIKLPAGTIAYLVNKDDNEDLRVLDLAIPVNGPDQLQSFLLSGSENQQSILSGFSKSVLEASFNTGYEEIE
KVLLEEREKETQHRRSLRDKRQHSQDEDVIVKLSRGQIEELSRNAKSSSKKSVSSESEPFNLRSRNPIYSNKFGKFFEITPEKNPQLQDLDVLVNSV
EIKEGSLLLPHYNSRAIVIVTVNDGKGDFEIVGQRNENRQGQRKEDDEEEEQGDENTNTQVQNYKAKLSRGDVFVIPAGHPVSIKASSNLDLLG
FGINAKNNQRNFLAGEEDNVISQIDRPVKELAFPGSAQEVDRLLENQKQSHFANAQPQQRERGSHETRDHLSSILDAF [SEQ ID 42]
   >gi|357507721|ref|XP_003624149.1| Provicilin [Medicago truncatula] >gi|87162569|gb|ABD28364.1| Cupin, RmlC-type [Medicagotruncatula] >gi|355499164|gb|AES80367.1|vicilin 47 kDa protein [Medicago truncatula] 
MAIKAPFQLLMLLGIFFLASVCVSSRDDRHDQENPFFFNANHFQTLFENENGHIRLLQRFDKRSKIFENLQNYRLLEYHSKPHTLFLPQHNDAD
FILAVLSGKAILTVLNPDNRNSFNLERGDTIKLPAGSIAYLANRDDNEDLRVLDLAIPVNRPGKFQSFSLSGSQNQQSFFSGFSKNILEAAFNANY
EEIERVLIEEHEQEPQHRRGLRKDRRQQSQDSNVIVKVSREQIEELSRHAKSSSRRSGSSESAPFNLRSREPIYSNEFGNFFEITPEKNPQLKDLDIL
VNYAEIREGSLLLPHFNSRATVIVVVDEGKGEFELVGQRNENQQEQREEDEQQEERSQQVQRYRARLSPGDVYVIPAGHPTVVSASSDLSLL
GFGINAENNERNFLAGEEDNVISQIERPVKEVAFPGSAQDVESLLKNQRQSYFANAQPQQREREEGRSQRQRELISSILGVF [SEQ ID 43]
P14323 (Rice)
   >gi|573918992|ref|XP_006647120.1| PREDICTED: glutelin type-B 2-like [Oryza brachyantha] 
MATTVFSRFSTYFCVLLLCHGSMAQLFNPSTNPWHNPRQGSSRECRFDRLQPFEPLRKVRSEAGVTEYFDEKNELFQCTGTFVIRRVIQPQGLL
VPRYTNAPGLVYIIQGRGSIGLTFPGCPATYQQQFQQFLPQEQSQSQKFRDEHQKIHQFRQGDIVALPAGVAHWFYNDGDAPVVAVYVYDV
KNSANQLEPRQREFLLGGNNMRAQQVYGSSAEQHSRQNIFSGFGVEILSEALGISTVTTKRLQSQNDQRGEIIHVKNGLQFLKPTLTQQQEQA
QAQYQEVQYSEQQQTSSRWNGLDENFCTIKARMNIENTSRADTYNPRAGRTTSLNSQKFPILNLVQMSATRVNLYQNAILSTFWNVNAHSL
VYTIQGRARVQVVSNFGKTVFDGELRPGQLLIIPQHYVVLKKAQREGFRYIAIKTNANAFVSQLVGKNSVFRSLPVDVIANVYRISREQARSLKN
NRGEEHGAFAPRSQQQSYPGFSNQSESETSE [SEQ ID 44]
   >gi|573919041|ref|XP_006647142.1| PREDICTED: glutelin type-B 4-like [Oryza brachyantha] 
MATTTFSRFSIYFCVLLLCHGSMAQLFSPTLNPWHSSRRGGSRDCRFDRLQAFEPLRRVRSEAGVTEYFDERNEQFQCTGTFVIRRVIEPQGLL
VPRYTNTPGVVYIMQGRGSMGLTFPGCPATYQQQFQQFLPEGQSQSQKFRDEHQKIHQFRQGDIVALPAGVAHWFYNEGDTPVVALYVFD
INNSANQLEPRQKDFLLAGNNNREQQVYGRSIEKHSGQNIFSGFNHELLSEALGISTLAAKRLQGQNDHRGEIIRVRNGLQLLKPTFTQQQEQ
AQSQYQVQYSEKQQESTRCNGLDENFCTINARLNIENPSRADTYNPRAGRITHLNNQKFPILNLVQMSATRVNLYQNAILSPYWNVNAHSLV
YMVQGHARVQVVSNLGKTVFNSVLRPGQLLIIPQHYVVLKKAEREGCQYIAFKTNANSIVSQLAGKNSILRAMPVDVVANAYRISREQARDLK
NNRGEELGAFTPKFEQQSYPGLSNESESEASE [SEQ ID 45]
   >gi|109894635|gb|ABG47337.1| glutelin precursor [Zizania latifolia] 
MNMATINGPTIFFTVCLFLLCHGSLAQLLGQSTSQWQSSHRGSSRQCRFDRLQAFEPVRSVRSQAGTTEFFDASNELFQCAGVSIVRRIIEPRG
LLLPQYTNGATIMYIIQGRGITGQTFPGCPESYQQQFQQSMQAQLTGSQSQSQKFKDEHQKINRFRQGDVIALPAGVAHWCYNDGEVPVVA
IYVIDINNAANQLDPRQRDFLLAGNMRSPQAYRREVENQSQNIFSGFSAELLSEALGISTGVARQLQCQNDQRGEIVRVEHGLSLLQPYASLQE
QEQKQEQPRERYQVTQHQQSQYGGGCSNGLDETFCAMRIWQNIDNPNLADTYNPRAGRVTNLNSQKFPILNLIQMSAVKVNLYQNALLSP
FWNINSHSVVYVTQGCARVQVVNNNGKTVFNGELRRGQLLIIPQHYVVVKKAQREGCAYIAFKTNPNSMVSHIVGKSSIFRALPTDVLANAY
RISREDAQRLKHNRGDELGAFTPLQYKSYQDVSSVAASS [SEQ ID 46]
P14614 (Rice)
   >gi|115445309|ref| NP_001046434.1| Os02g0248800 [Oryza sativa Japonica Group] >gi||37993738|gb|AAR06952.1| glutelin type-B [Oryza sativa Japonica Group] >gi|447477729|dbj|BAD19794.1| glutelin type-B [Oryza sativa Japonica Group] >gi|113535965|dbj|BAF08348.1| Os02g0248800 [Oryza sativa Japonica Group] >gi|215768942|dbj|BAH01171.1| unnamed protein product [Oryza sativa Japonica Group] >gi|284431772|gb|ADB84627.1| glutelin [Oryza sativa Japonica Group] 
MTISVFSRFSIYFCVLLLCNGSMAQLFDPATNQWQTHRQGSFRECRFERLQAFEPLQNVRSEAGVTEYFDETNELFQCTGTFVIRRVIQPQGLL
IPRYANTPGMVYIIQGRGSMGLTFPGCPATYQQQSQQFLFQGESQSQKFIDEHQKIHQFRQGDIVVLPTGVAHWFYNDGDTPVVALYVYDI
NNSANQLEPRHREFLLAGKNNRVQQVYGRSIQQHSGQNIFNGFSVEPLSEALNINTVTTKRLQSQNDQRGEIIHVKNGLQLLKPTLTQRQEQE
QAQYQEVQYSEKPQTSSRWNGLEENLCTIKTRLNIENPSRADSYDPRAGRITSLDSQKFPILNIIQMSATRVNLYQNAILTPFWNVNAHSLMYV
IRGRARVQVVSNFGKTVFDGVLRPEQLLIIPQNYVVLKKAQHEGCQYIAINTNANAFVSHLAGVDSVFHALPVDVIANAYCISREEARRLKNNR
GDEYGPFPPRLQQQIYPEFSNESKGETSE [SEQ ID 47]
   >gi|428674402|gb|AFZ41188.1| glutelin, partial [Oryza sativa Japonica Group] 
LLCHGSMAQIFSLGINPWQNPRQGGSRECRFDRLQAFEPLRKVRHEAGVTEYFDEKNEQFQCTGTLVIRRIIEPQGLLLPRYSNTPGLVYIIQGT
GVLGLTFPGCPATYQKQFRHFGLEGGSQRQGKKLRDENQKIHQFRQGDVVALPSGIPHWFYNEGDTPVVALFVFDVNNNANQLEPRQKEFL
LAGNNIEQQVSNPSINKHSGQNIFNGFNTKLLSEALGVNIEVTRRLQSQNDRRGDIIRVKNGLRLIKPTITQQQEQTQDQYQQIQYHREQRSTS
KYNGLDENFCAIRARLNIENPNHADTYNPRAGRITNLNSQKFSILNLVQMSATRVNLYQNAILSPFWNINAHSLVYTIQGRARVQVVSNHGKA
VFNGVLRPGQLLIIPQNYVVMKKAELEGFQFIAFKTNPNAMVNHIAGKNSVLRAMPVDVIANAYRISRQEARSLKNNRGEEIGAFTPRYQQQ
KIHQEYSNPNESETQ [SEQ ID 48]
   >gi|226510|prf||1515394A seed storage globulin 
MATTRFPSLLFYSCIFLLCNGSMAQLFGQSFTPWQSSRQGGLRGCRFDRLQAFEPLRQVRSQAGITEYFDEQNEQFRCAGVSVIRRVIEPQGLL
LPQYHNAPGLVYILQGRGFTGLTFPGCPATFQQQFQPFDQARFAQGQSKSQNLKDEHQRVHHIKQGDVVALPAGIVHWCYNDGDAPIVAV
YVFDVNNNANQLEPRQKEFLLAGNNKREQQFGQNIFSGFSVQLLSEALGISQQAAQKIQSQNDQRGEIIRVSQGLQFLKPFVSQQGPVEHQA
YQPIQSQQEQSTQYQVGQSPQYQEGQSTQYQSGQSWDQSFNGLEENFCSLEARQNIENPKRADTYNPRAGRITHLNSKNFPTLNLVQMSA
TRVNLYQNAILSPYWNINAHSVMHMIQGRARVQVVNNHGQTVFNDILRRGQLLIIPQHYVVLKKAEREGCQYISFKTTPNSMVSYIAGKTSIL
RALPVDVLANAYRISRQESQNLKNNRGEEFGAFTPKFAQTGSQSYQDEGESSSTEKASE [SEQ ID 49]
P07728 (Rice)
   >gi|531874314|gb|AGT59174.1| glutelin, partial [Oryza sativa Indica Group] 
CRFDRLQAFEPIRSVRSQAGTTEFFDVSNEQFQCTGVSAVRRVIEPRGLLLPHYTNGASLVYIIQGRGITGPTFPGCPESYQQQFQQSGQAQLT
ESQSQSHKFKDEHQKIHRFRQGDVIALPAGVAHWCYNDGEVPVVAIYVTDLNNGANQLDPRQRDFLLAGNKRNPQAYRREVEERSQNIFSG
FSTELLSEALGVSSQVARQLQCQNDQRGEIVRVEHGLSLLQPYASLQEQEQGQVQSRERYQEGQYQQSQYGSGCSNGLDETFCTMKVRQNI
DNPNRADTYNPRAGRVTNLNTQNFPILNLVQMSAVKVNLYQNALLSPFWNINAHSVVYITQGRARVQVVNNNGKTVFNGELRRGQLLIIPQ
HYAVVKKAQREGCAYIAFKTNPNSMVSHIAGKSSIFRALPNDVLANAYRISREEAQRLKHNRGDEFGAFTPIQYKSYQDVYNAAESS [SEQ ID
50]
   >gi|09894635|gb|ABG47337.1| glutelin precursor [Zizania latifolia] 
MNMATINGPTIFFTVCLFLLCHGSLAQLLGQSTSQWQSSHRGSSRQCRFDRLQAFEPVRSVRSQAGTTEFFDASNELFQCAGVSIVRRIIEPRG
LLLPQYTNGATIMYIIQGRGITGQTFPGCPESYQQQFQQSMQAQLTGSQSQSQKFKDEHQKINRFRQGDVIALPAGVAHWCYNDGEVPVVA
IYVIDINNAANQLDPRQRDFLLAGNMRSPQAYRREVENQSQNIFSGFSAELLSEALGISTGVARQLQCQNDQRGEIVRVEHGLSLLQPYASLQE
QEQKQEQPRERYQVTQHQQSQYGGGCSNGLDETFCAMRIWQNIDNPNLADTYNPRAGRVTNLNSQKFPILNLIQMSAVKVNLYQNALLSP
FWNINSHSVVYVTQGCARVQVVNNNGKTVFNGELRRGQLLIIPQHYVVVKKAQREGCAYIAFKTNPNSMVSHIVGKSSIFRALPTDVLANAY
RISREDAQRLKHNRGDELGAFTPLQYKSYQDVSSVAASS [SEQ ID 51]
   >gi|472867|emb|CAA52764.1| 11S globulin [Avena sativa]

MATTSFPSMLFYFCIFLLFHGSMAQLFGQSSTPWQSSRQGGLRGCRFDRLQAFEPLRQVRSQAGITEYFDEQNEQFRCTGVSVIRRVIEPQGL
VLPQYHNAPALVYILQGRGFTGLTFPGCPATFQQQFQPFDQSQFAQGQRQSQTIKDEHQRVQRFKQGDVVALPAGIVHWCYNDGDAPIVA
IYVFDVNNNANQLEPRQKEFLLAGNNKREQQSGNNIFSGLSVQLLSEALGISQQAAQRIQSQNDQRGEIIRVSQGLQFLKPIVSQQVPGEQQV
YQPIQTQEGQATQYQVGQSTQYQVGKSTPYQGGQSSQYQAGQSWDQSFNGLEENFCSLEARKNIENPQHADTYNPRAGRITRLNSKNFPIL
NIVQMSATRVNLYQNAILSPFWNINAHSVIYMIQGHARVQVVNNNGQTVFNDILRRGQLLIVPQHFVVLKKAEREGCQYISFKTNPNSMVSH
IAGKSSILRALPIDVLANAYRISRQEARNLKNNRGEEFGAFTPKLTQKGFQSYQDIEEGSSSPVRASE [SEQ ID 52]

## Claims

1. A natural antibacterial peptide comprising an antibacterial fragment of a protein selected from SEQUENCE ID NO's: 1 to 6, or an antibacterial variant of the fragment, in which the antibacterial fragment is selected from SEQUENCE ID NO's: 7 to 35, wherein:
the antibacterial fragment has between 8 and 13 amino acids;
the c-terminal amino acid of the antibacterial fragment is not C, F, I, K, M, P, Q, R, T, V, W; and
the n-terminal amino acid of the antibacterial fragment is not C, D, H, K, M, P, Q, T, V, W.

2. A natural antibacterial peptide of Claim 1 wherein the protein is a pea protein SEQUENCE ID NO's: 1 to 3, and wherein the antibacterial fragment is selected from SEQUENCE ID NO's: 7-25.

3. A natural antibacterial peptide of Claim 1 wherein the protein is a rice protein selected from SEQUENCE ID NO's: 4 to 6, and wherein the antibacterial fragment is selected from SEQUENCE ID NO's:26-35.

4. A natural antibacterial peptide of Claim 1 wherein the protein is a rice protein of SEQUENCE ID NO's: 4.

5. A natural antibacterial peptide of Claim 1 wherein the protein is a rice protein of SEQUENCE ID NO's: 4 and in which the antibacterial fragment is SEQUENCE ID NO: 32 or an antibacterial variant of the fragment.

6. A composition comprising at least one antibacterial peptide of Claim 2.

7. A composition according to Claim 6, and including a peptide profile comprising substantially all of the antibacterial fragments of SEQUENCE ID NO's: 7-25.

8. A composition comprising at least one antibacterial peptide of Claim 3.

9. A composition according to Claim 8 and including a peptide profile comprising substantially all of the antibacterial fragments of SEQUENCE ID NO's: 26-35.

10. A comestible product for human or animal consumption comprising a composition according to any of Claims 6 to 9.

11. A cleaning product comprising a composition according to any of Claims 6 to 9.

12. A plant biocide comprising a composition according to any of Claims 6 to 9.

13. Use of a composition according to any of Claims 6 to 9 as a preservative.

14. Use of a composition according to any of Claims 6 to 9 as a plant biocide.

15. A natural antibacterial peptide of any of Claims 1 to 5, or composition of any of Claims 6 to 9, **for use in** treating a disease or condition **characterised by** a pathogenic bacterial infection.
